# EUROPEAN PATENT SPECIFICATION

(11) **EP 0 765 873 B1**
(45) Date of publication and mention of the grant of the patent: **17.04.2002**
(21) Application number: 96118182.3
(22) Date of filing: 13.10.1992
(51) Int. Cl.: C07D 239/10, C07D 245/02, C07D 401/14, C07D 403/14, C07D 405/14, C07D 409/14, A61K 31/505, A61K 31/395, C07D 243/04, C07D 413/14, C07D 413/06, C07D 401/06

(54) **Cyclic ureas and analogues useful as retroviral protease inhibitors**
Cyclische Harnstoffe und Analoga verwendbar als retrovirale Proteasehemmer
Urées cycliques et analogues utiles comme inhibiteurs de protéase rétrovirale

(30) Priority: 11.10.1991 US 776491; 15.05.1992 US 883944; 29.09.1992 US 953272
(43) Date of publication of application: 02.04.1997
(62) Divisional of application: 92922262.8
(73) Proprietor: DuPont Pharmaceuticals Company, Wilmington, DE 19805 (US)
(72) Inventor: Lam, Patrick Yuk-Sun, Chadds Ford, PA 19317 (US); Eyermann, Charles Joseph, Wilmington, Delaware 19809 (US); Hodge, Carl Nicholas, Wilmington, Delaware 19803 (US); Jadhav, Prabhakar Kondaji, Wilmington, Delaware 19808 (US); DeLucca, George Vincent, Wilmington, Delaware 19810 (US)
(74) Representative: von Kreisler, Alek, Dipl.-Chem.

(56) References cited:
- JOURNAL OF ORGANIC CHEMISTRY, vol. 25, no. 9, 7 September 1960, WASHINGTON DC, US, pages 1664-1665, XP002023590 P.F. WILEY ET AL.: "Synthesis of 2,4-dimethyl-3-hydroxy-6-oxoheptanal"
- JOURNAL OF ORGANIC CHEMISTRY, vol. 27, no. 8, 7 August 1962, WASHINGTON DC, US, pages 2828-2832, XP002023591 S. SEARLES JR. ET AL.: "Synthesis of cyclic sulfides from cyclic carbonate esters. I. Thietanes"
- JOURNAL OF CHEMICAL SOCIETY, PERKIN TRANSACTIONS I, no. 7, 1983, LONDON GB,, pages 1387-1393, XP002023592 R. BAKER ET AL.: "Stereoselective total synthesis of racemic (3S,4R/3R,4S)- and a diastereoisomeric mixture of (6E,10Z) -3,4,7,11-tetramethyl-trideca-6,10-dienal (Faranal); the trail pheromone of the Pharaoh's ant"
- BULLETIN DE LA SOCI T CHIMIQUE DE FRANCE, 1966, PARIS, FR, pages 2005-2011, XP002023599 J. COLONGE ET AL.: "Sur la préparation des alcoyltétrahydropyrannols-2"
- TETRAHEDRON LETTERS, vol. 25, no. 39, 1984, OXFORD, GB, pages 4371-4374, XP002023600 C. SANTELLI-ROUVIER: "Obtention de la lactone de Prelog-Djerassi et d'autres delta-lactones par addition du pentényltriméthylsilane sur des dérivés de l'anhydride glutarique"
- TETRAHEDRON LETTERS vol. 22, no. 42, 1981, OXFORD GB, +-) pages 4235 - 4238, XP002023601 K. MARUYAMA ET AL.: 'A short and stereoselective synthesis of the (+-) Prelog-Djerassi lactonic acid'
- JOURNAL OF ORGANIC CHEMISTRY vol. 54, no. 1, 6 January 1989, WASHINGTON DC, US, pages 11-12, XP002023602 M. EGBERTSON ET AL. : "Synthetic route to the "tricarbonyl" region of FK-506"
- CHIMICA THERAPEUTICA vol. III, no. 5, 1968, PARIS, FR, pages 321 - 324, XP002023603 J. MAILLARD ET AL.: 'Composés cycloalcane spiro-hétérocycliques. III. Synthèse de tétrahydro(2H) oxazine-1,3 ones-2 disubstituées en -6'
- MONATSHEFTE FÜR CHEMIE vol. 92, no. 1, 28 February 1961, WIEN, AT, pages 79 - 87, XP002023604 G. ZIGEUNER ET AL.: 'Über Heterocyclen, 3. Mitt.: Zur Reaktion von Homologen des Acetaldehyds mit Carbamiden'
- TETRAHEDRON LETTERS vol. 30, no. 10, 1989, OXFORD, GB, pages 1253 - 1256, XP002023605 T. SHONO ET AL.: 'Diastereoselectivity in the addition of enolate anions to N-methoxycarbonylimines generated in situ from alpha-methoxy carbamates'
- JOURNAL OF ORGANIC CHEMISTRY vol. 56, no. 1, 04 January 1991, WASHINGTON DC, US, pages 365 - 372, XP002023606 P.G.M. WUTS ET AL.: 'The addition of gamma-(trimethylsilyl)allylboronates to imines'
- JOURNAL OF ORGANIC CHEMISTRY vol. 44, no. 9, 27 April 1979, WASHINGTON DC, US, pages 1391 - 1397, XP002023607 J.A. MARSHALL: 'Stereoselective synthesis of racemic elemanolide dilactones related to vernolepin'
- LIEBIGS ANNALEN DER CHEMIE no. 12, 12 February 1985, WEINHEIM, DE, pages 275 - 300, XP002023608 B. BARDILI ET AL.: 'Bildung und Reaktivität von hydroxysubstituierten gamma- und delta-Lactonen'
- TETRAHEDRON LETTERS vol. 28, no. 27, 1987, OXFORD, GB, pages 3103 - 3106, XP002023609 D. SEEBACH ET AL.: 'Alpha-alkylation of beta-aminobutanoates with Ik-1.2-induction'
- TETRAHEDRON LETTERS vol. 28, no. 43, 1987, OXFORD, GB, pages 5129 - 5132, XP002023610 M.D. LEWIS ET AL.: 'Absolute and relative configuration of L-660,631'
- JOURNAL OF ORGANIC CHEMISTRY vol. 47, no. 7, 26 March 1982, WASHINGTON DC, US, pages 1371 - 1373, XP002023611 W.R. ROUSH ET AL.: 'Total synthesis of carbohydrates: stereoselective synthesis of 2,6-dideoxy-D-arabino-hexose and 2,6-dideoxy-D-ribo-hexose'
- JOURNAL OF MEDICINAL CHEMISTRY vol. 33, no. 10, October 1990, WASHINGTON DC, US, pages 2687 - 2689, XP002023613 D.J. KEMPF ET AL.: 'Structure-based, C2 symmetric inhibitors of HIV protease'
- ZIGEUNER G ET AL: 'Über Heterocyclen, 5. Mitt.: 1-Oxo-2,10a-diaza-9-oxaoctahydrophenanthren e' MONATSHEFTE FÜR CHEMIE vol. 96, no. 6, 1965, pages 1943 - 1949
- ZIGEUNER G ET AL: 'Über Heterocyclen, 6. Mitt.: Cyclische Harnstoff - Aldehyd-Kondensate' MONATSHEFTE FÜR CHEMIE vol. 96, no. 6, 1965, pages 1950 - 1966

## Description

### FIELD OF THE INVENTION

This invention relates to substituted cyclic carbonyls and derivatives thereof useful as retroviral protease inhibitors, to pharmaceutical compositions comprising such compounds, and to methods of using these compounds for treating viral infection.

### BACKGROUND OF THE INVENTION

Current treatments for viral diseases usually involve administration of compounds that inhibit viral DNA synthesis. Current treatments for AIDS (Dagani, *Chem. Eng. News*, November 23, 1987 pp. 41-49) involve administration of compounds such as 2',3'-dideoxycytidine, trisodium phosphonoformate, ammonium 21-tungsto-9-antimoniate, 1-b-D-ribofuranoxyl-1,2,4-triazole-3-carboxamide, 3'-azido-3'-deoxythymidine (AZT), and adriamycin that inhibit viral DNA synthesis; compounds such as AL-721 and polymannoacetate which may prevent HIV from penetrating the host cell; and compounds which treat the opportunistic infections caused by the immunosupression resulting from HIV infection. None of the current AIDS treatments have proven to be totally effective in treating and/or reversing the disease. In addition, many of the compounds currently used to treat AIDS cause adverse side effects including low platelet count, renal toxicity, and bone marrow cytopenia.

Proteases are enzymes which cleave proteins at specific peptide bonds. Many biological functions are controlled or mediated by proteases and their complementary protease inhibitors. For example, the protease renin cleaves the peptide angiotensinogen to produce the peptide angiotensin I. Angiotensin I is further cleaved by the protease angiotensin converting enzyme (ACE) to form the hypotensive peptide angiotensin II. Inhibitors of renin and ACE are known to reduce high blood pressure *in vivo.* However, no therapeutically useful renin protease inhibitors have been developed, due to problems of oral availability and *in vivo* stability.

The genomes of retroviruses encode a protease that is responsible for the proteolytic processing of one or more polyprotein precursors such as the pol and gag gene products. See Wellink, *Arch. Virol.* 98 1 (1988). Retroviral proteases most commonly process the gag precursor into the core proteins, and also process the pol precursor into reverse transcriptase and retroviral protease.

The correct processing of the precursor polyproteins by the retroviral protease is necessary for the assembly of the infectious virions. It has been shown that *in vitro* mutagenesis that produces protease-defective virus leads to the production of immature core forms which lack infectivity. See Crawford, J. *Virol.* 53 899 (1985); Katoh et al., *Virology* 145 280 (1985). Therefore, retroviral protease inhibition provides an attractive target for antiviral therapy. See Mitsuya, *Nature* 325 775 (1987).

Moore, *Biochem. Biophys. Res. Commun*., 159 420 (1989) discloses peptidyl inhibitors of HIV protease. Erickson, European Patent Application No. WO 89/10752 discloses derivatives of peptides which are inhibitors of HIV protease.

U.S. Patent No. 4,652,552 discloses methyl ketone derivatives of tetrapeptides as inhibitors of viral proteases. U.S. Patent No. 4,644,055 discloses halomethyl derivatives of peptides as inhibitors of viral proteases. European Patent Application No. WO 87/07836 discloses L-glutamic acid gamma-monohydroxamate as an antiviral agent.

The ability to inhibit a viral protease provides a method for blocking viral replication and therefore a treatment for viral diseases, such as AIDS, that may have fewer side effects, be more efficacious, and be less prone to drug resistance when compared to current treatments.

The topic of the present invention is substituted cyclic carbonyls and derivatives thereof, which compounds are capable of inhibiting viral protease and which compounds are believed to serve as a means of combating viral diseases, such as AIDS. The substituted cyclic carbonyls and derivatives thereof of this invention provide significant improvements over protease inhibitors that are known in the art. A large number of compounds have been reported to be inhibitors of proteases, such as renin, but these have suffered from lack of adequate bioavailability and are thus not useful as therapeutic agents, particularly if oral administration is desired. This poor activity has been ascribed to the relatively high molecular weight of most protease inhibitors, to inadequate solubility properties, and to the presence of a number of peptide bonds, which are vulnerable to cleavage by mammalian proteases in vivo and which generally cause the molecules to be extensively bound in human serum. The substituted cyclic carbonyls and derivatives described herein have a distinct advantage in this regard, in that they do not contain peptide bonds, are of low molecular weight, and can be hydrophilic yet still inhibit the viral protease enzyme.

Additionally, known inhibitors of other non-HIV proteases do not inhibit HIV protease. The structure-activity requirements of such inhibitors differ from those of HIV protease inhibitors. The substituted cyclic carbonyls and derivatives of the invention are particularly useful as inhibitors of HIV protease and similar retroviral proteases.

Other HIV protease inhibitors have been reported, but to date none have been shown to be clinically effective. This lack of utility is due in part to the factors discussed above for renin inhibitors, particularly low bioavailability. The compounds of the invention offer a valuable solution to this problem in that they are of low molecular weight and many, therefore, have good oral absorption properties in mammals, ranging from 1-100% absolute oral availability.

### DETAILED DESCRIPTION OF THE INVENTION

There is provided by this invention a compound of the formula (I): 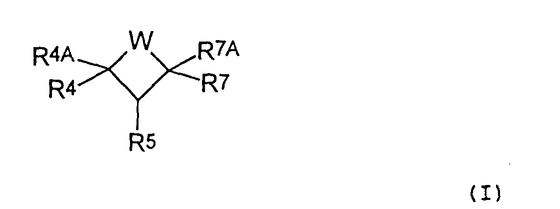 or a pharmaceutically acceptable salt or prodrug form thereof wherein:
R⁴ and R⁷ are independently selected from the following groups:
   hydrogen;
   C₁-C₈ alkyl substituted with 0-3 R¹¹;
   C₂-C₈ alkenyl substituted with 0-3 R¹¹;
   C₂-C₈ alkynyl substituted with 0-3 R¹¹;
   C₃-C₈ cycloalkyl substituted with 0-3 R¹¹;
   C₆-C₁₀ bicycloalkyl substituted with 0-3 R¹¹;
   aryl substituted with 0-3 R¹²;
   a C₆-C₁₄ carbocyclic residue substituted with 0-3 R¹²;
   a heterocyclic ring system substituted with 0-2 R¹², composed of 5 to 10 atoms including at least one nitrogen, oxygen or sulfur atom;
R^{4A} and R^{7A} are independently selected from the following groups:
   hydrogen;
   C₁-C₄ alkyl substituted with halogen or C₁-C₂ alkoxy;
   benzyl substituted with halogen or C₁-C₂ alkoxy;
R⁴ and R^{4A} can alternatively join to form a 5-7 membered carbocyclic ring substituted with 0-2 R¹²;
R⁷ and R^{7A} can alternatively join to form a 5-7 membered carbocyclic ring substituted with 0-2 R¹²;
R⁵ is selected from fluoro, difluoro, =O, and -OR²⁰;
R²⁰ is selected from:
   hydrogen;
   C₁-C₆ alkyl substituted with 0-3 R¹¹;
   C₃-C₆ alkoxyalkyl substituted with 0-3 R¹¹;
   C₁-C₆ alkylcarbonyl substituted with 0-3 R¹¹;
   C₁-C₆ alkoxycarbonyl substituted with 0-3 R¹¹;
   benzoyl substituted with 0-3 R¹²;
   phenoxycarbonyl substituted with 0-3 R¹²;
   phenylaminocarbonyl substituted with 0-3 R¹²;
R¹¹ is selected from one or more of the following:
   keto, halogen, cyano, -CH₂NR¹³R¹⁴, -NR¹³R¹⁴, -CO₂R¹³, -OC(=O)R¹³, -OR¹³, C₂-C₆ alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
   1-3 amino acids, linked together via amide bonds and linked to R⁴ or R⁷ via the amine or carboxylate terminus;
   a C₅-C₁₄ carbocyclic residue substituted with 0-3 R¹²;
   aryl substituted with 0-3 R¹²;
   a heterocyclic ring system substituted with 0-2 R¹², composed of 5 to 10 atoms including at least one nitrogen, oxygen or sulfur atom;
R¹², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy;
   a 5- or 6-membered heterocyclic ring containing from 1 to 4 heteroatoms selected from oxygen, nitrogen and sulfur;
   or R¹² may be a 3- or 4- carbon chain attached to adjacent carbons on the ring to form a fused 5- or 6- membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl;
R¹³ is H, phenyl, benzyl, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-,
-(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is selected from:
   -N(R²²)C(=Z)N(R²³)-;
   -N(R²²)S(=O)N(R²³)-;
   wherein:
Z is O, S, or NR²⁴;
R²² and R²³ are independently selected from the following:
   hydrogen;
   C₁-C₈ alkyl substituted with 0-3 R³¹;
   C₂-C₈ alkenyl substituted with 0-3 R³¹;
   C₂-C₈ alkynyl substituted with 0-3 R³¹;
   C₃-C₈ cycloalkyl substituted with 0-3 R³¹;
   C₆-C₁₀ bicycloalkyl substituted with 0-3 R³¹;
   aryl substituted with 0-3 R³²;
   a C₆-C₁₄ carbocyclic residue substituted with 0-3 R³²;
   a heterocyclic ring system substituted with 0-2 R³², composed of 5 to 10 atoms including at least one nitrogen, oxygen or sulfur atom;
R²⁴ is selected from: hydroxy; amino; C₁-C₄ alkyl; C₁-C₄ alkoxy; C₁-C₄ aminoalkyl; cyano; nitro; benzyloxy;
alternatively, R²² can join with R⁴ or R^{4A} to form a five- or six-membered fused heterocyclic or carbocyclic ring substituted with 0-2 R¹²; and
alternatively, R²³ can join with R⁷ or R^{7A} to form a five- or six-membered fused heterocyclic or carbocyclic ring substituted with 0-2 R¹²; and
alternatively, W can join with R⁵ to form a three-to seven-membered fused heterocyclic or carbocyclic ring substituted with 0-2 R¹²;
R³¹ is selected from one or more of the following:
   keto, halogen, cyano, -CH₂NR¹³R¹⁴, -NR¹³R¹⁴, -CO₂R¹³, -OC(=O)R¹³, -OR¹³, C₂-C₆ alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
   1-3 amino acids, linked together via amide bonds and linked to R⁴ or R⁷ via the amine or carboxylate terminus;
   a C₅-C₁₄ carbocyclic residue substituted with 0-3 R³²;
   aryl substituted with 0-3 R³²; or
   a heterocyclic ring system substituted with 0-2 R³², composed of 5 to 10 atoms including at least one nitrogen, oxygen or sulfur atom;
R³², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy, -C(R¹⁴)=N(OR¹⁴) ; or
   a 5- or 6-membered heterocyclic ring containing from 1 to 4 heteroatoms selected from oxygen, nitrogen and sulfur;
   or R³² may be a 3- or. 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or
   -NR¹³R¹⁴; or, when R³² is attached to a saturated carbon atom, it may be carbonyl or thiocarbonyl;
R³², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl, -C(R¹⁴)=N(OR¹⁴);
   provided that:
   R⁴, R^{4A}, R⁷, and, R^{7A} are not all hydrogen;
   when R⁴ and R^{4A} are both hydrogen, at least one of R²² and R²³ is not hydrogen.

Preferred compounds of this invention are compounds wherein:
R⁴ and R⁷ are independently selected from the following groups:
   hydrogen;
   C₁-C₄ alkyl substituted with 0-3 R¹¹;
   C₃-C₄ alkenyl substituted with 0-3 R¹¹;
   C₃-C₄ alkynyl substituted with 0-3 R¹¹;
R^{4A} and R^{7A} are hydrogen;
R²⁰ is selected from:
   hydrogen;
   C1-C6 alkylcarbonyl;
   C₁-C₆ alkoxycarbonyl;
   benzoyl;
R¹¹ is selected from one or more of the following:
   keto, halogen, cyano, -CH₂NR¹³R¹⁴, -NR¹³R¹⁴, -CO₂R¹³,
      -OC(=O)R¹³, -OR¹³, C₂-C₄ alkoxyalkyl, -S(O)ₘR¹³, C₁-C₄ alkyl, C₂-C₄ alkenyl, C₃-C₆ cycloalkyl;
   a C₅-C₁₄ carbocyclic residue substituted with 0-3 R¹²;
   aryl substituted with 0-3 R¹²;
   a heterocyclic ring system substituted with 0-2 R¹², composed of 5 to 10 atoms including at least one nitrogen, oxygen or sulfur atom;
R¹², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴; or
   a 5- or 6-membered heterocyclic ring containing from 1 to 4 heteroatoms selected from oxygen, nitrogen or sulfur;
   or R¹² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H;
R¹³ is H, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is
   -N(R²²)C(=Z)N(R²³)-;
   wherein:
Z is O, S, N-CN, N-OH, N-OCH₃;
R²² and R²³ are independently selected from the following:
   hydrogen;
   C₁-C₈ alkyl substituted with 0-3 R³¹;
   C₃-C₈ alkenyl substituted with 0-3 R³¹;
   C₃-C₈ alkynyl substituted with 0-3 R³¹;
   C₃-C₆ cycloalkyl substituted with 0-3 R³¹;
R³¹ is selected from one or more of the following:
   keto, halogen, cyano, -CH₂NR¹³R¹⁴, -NR¹³R¹⁴, -CO₂R¹³, -OC(=O)R¹³, -OR¹³, C₂-C₄ alkoxyalkyl, -S(O)ₘR¹³, C₁-C₄ alkyl, C₂-C₄ alkenyl, C₃-C₆ cycloalkyl;
   a C₅-C₁₄ carbocyclic residue substituted with 0-3 R¹²;
   aryl substituted with 0-3 R³²;
   a heterocyclic ring system substituted with 0-2 R³², composed of 5 to 10 atoms including at least one nitrogen, oxygen or sulfur atom;
R³², when a substituent on carbon, is selected from one or more of the following:
   phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -C(R¹⁴)=N(OR¹⁴);
   a 5- or 6-membered heterocyclic ring containing from 1 to 4 heteroatoms selected from oxygen, nitrogen and sulfur;
   or R³² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R³² is attached to a saturated carbon atom, it may be carbonyl or thiocarbonyl;
R³², when a substituent on nitrogen, is selected from one or more of the following:
   phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H, -C(R¹⁴)=N(OR¹⁴);
   provided that:
   R⁴, R^{4A}, R⁷, and R^{7A} are not all hydrogen;
   when R⁴ and R^{4A} are both hydrogen, at least one of R²² and R²³ is not hydrogen.

Further preferred compounds of the invention of formula (I) are compounds of formula (II) : 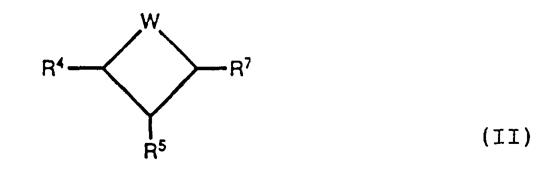 or a pharmaceutically acceptable salt or prodrug form thereof wherein:
R⁴ and R⁷ are independently selected from the following groups:
   hydrogen;
   C₁-C₄ alkyl substituted with 0-3 R¹¹;
   C₃-C₄ alkenyl substituted with 0-3 R¹¹;
R⁵ is -OR²⁰;
R²⁰ is as defined in claim 1;
R¹¹ is selected from one or more of the following:
   keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
   aryl substituted with 0-3 R¹²;
   a heterocyclic ring system substituted with 0-2 R¹², composed of 5 to 10 atoms including at least one nitrogen, oxygen or sulfur atom;
R¹², when a substituent on carbon, is selected from one or more of the following:
   phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, methylenedioxy, C₁-C₄ haloalkyl, C₁-C₄ alkylcarbonyl, C₁-C₄ alkylcarbonylamino, hydroxy, hydroxymethyl; or
   a 5- or 6-membered heterocyclic ring containing from 1 to 4 heteroatoms selected from oxygen, nitrogen or sulfur;
R¹², when a substituent on nitrogen, is selected from benzyl or methyl;
R¹³ is H, C₁-C₂ alkyl, or C₃-C₆ alkoxyalkyl;
R¹⁴ is OH, H or C₁-C₂ alkyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
W is -N(R²²)C(=Z)N(R²³)-;
   wherein:
Z is O, S, or N-CN;
R²² and R²³ are independently selected from the following:
   hydrogen;
   C₁-C₄ alkyl substituted with 0-3 R³¹;
   C₃-C₄ alkenyl substituted with 0-3 R³¹;
R³¹ is selected from one or more of the following:
   keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
   aryl substituted with 0-3 R³²; or
   a heterocyclic ring system substituted with 0-2 R³², composed of 5 to 10 atoms including at least one nitrogen, oxygen or sulfur atom;
R³², when a substituent on carbon, is selected from one or more of the following:
   phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, methylenedioxy, C₁-C₄ haloalkyl, C₁-C₄ alkylcarbonyl, C₁-C₄ alkylcarbonylamino, hydroxy, hydroxymethyl, -C(R¹⁴)=N(OR¹⁴);
   a 5- or 6-membered heterocyclic ring containing from 1 to 4 heteroatoms selected from oxygen, nitrogen and sulfur;
R³², when a substituent on nitrogen, is benzyl or methyl;
   provided that:
   when R⁴ is hydrogen, R⁷ is not hydrogen;
   when R⁴ is hydrogen, at least one of R²² and R²³ is not hydrogen.

Also preferred are compounds of formula (III): 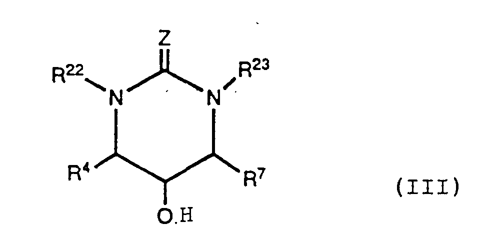 wherein:
R⁴ and R⁷ are independently selected from the following groups:
   hydrogen;
   C₁-C₃ alkyl substituted with 0-3 R¹¹;
R¹¹ is selected from one or more of the following:
   halogen, -OR¹³, C₁-C₄ alkyl, C₃-C₅ cycloalkyl;
   aryl substituted with 0-2 R¹²;
   a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R¹², when a substituent on carbon, is selected from one or more of the following:
   benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl; or
R¹², when a substituent on nitrogen, is methyl;
R¹³ is H or methyl;
R¹⁴ is OH, H or methyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R²² and R²³ are independently selected from the following:
   hydrogen;
   C₁-C₄ alkyl substituted with 0-1 R³¹;
   C₃-C₄ alkenyl substituted with 0-1 R³¹;
R³¹ is selected from one or more of the following:
   halogen, -OR¹³, C₁-C₄ alkyl, C₃-C₅ cycloalkyl;
   aryl substituted with 0-2 R³²;
   a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R³², when a substituent on carbon, is selected from one or more of the following:
   benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl, -C(R¹⁴)=N(OR¹⁴); or
R³², when a substituent on nitrogen, is methyl;
   provided that:
   when R⁴ is hydrogen, R⁷ is not hydrogen;
   when R⁴ is hydrogen, at least one of R²² and R²³ is not hydrogen.

Specifically preferred are compounds of formula (IV): 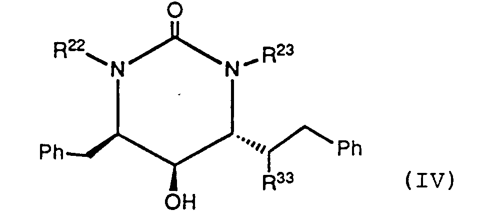 or a pharmaceutically acceptable salt or prodrug form thereof wherein:
R³³ is OH, halogen, H, N₃, or can alternatively be taken together with R²³ to form a direct bond;
R²² and R²³ are independently selected from:
   hydrogen, allyl, propyl, cyclopropylmethyl, n-butyl, i-butyl, CH₂CH=CH(CH₃)₂, pyridylmethyl, methallyl, n-pentyl, i-pentyl, hexyl, benzyl, pyridylmethyl, isoprenyl, propargyl, picolinyl, methoxyethyl, cyclohexylmethyl, dimethyl-butyl, ethoxyethyl, methyloxazolinylmethyl, naphthylmethyl, methyloxazolinylmethyl, vinyloxyethyl, pentafluorobenzyl, quinolinylmethyl, carboxybenzyl, chloro-thienyl, picolinyl, benzyloxybenzyl, phenylbenzyl, adamantylethyl, cyclopropylmethoxybenzyl, ethoxybenzyl, hydroxybenzyl, hydroxymethylbenzyl, aminobenzyl, formylbenzyl, cyanobenzyl, cinnamyl, allyloxybenzyl, fluorobenzyl, cyclobutylmethyl, formaldoximebenzyl, cyclopentylmethyl, nitrobenzyl, nitrilobenzyl, carboxamidobenzyl, carbomethoxybenzyl, and dimethylallyl.

It has been found that the compounds above are useful as inhibitors of HIV protease and similar retroviral proteases, and for the treatment of HIV infection and similar retrovirus infections.

The present invention also provides a pharmaceutical composition comprising the compounds of formulas (I)-(IV) as defined above and the use of the compounds of formulas (I)-(IV) as defined above for the preparation of a medicament for the treatment of viral infections such as HIV infection.

The compounds herein described may have asymmetric centers. All chiral, diastereomeric, and racemic forms are included in the present invention. Many geometric isomers of olefins and C=N double bonds can also be present in the compounds described herein, and all such stable isomers are contemplated in the present invention.

When any variable (for example, R¹ through R²⁸, R^{4A} and R^{7A}, m, n, W, Z, etc.) occurs more than one time in any constituent or in formula (I) to (IV) or any other formula herein, its definition on each occurrence is independent of its definition at every other occurrence. Also, combinations of substituents and/or variables are permissible only if such combinations result in stable compounds.

As used herein, "alkyl" is intended to include both branched and straight-chain saturated aliphatic hydrocarbon groups having the specified number of carbon atoms; "alkoxy" represents an alkyl group of indicated number of carbon atoms attached through an oxygen bridge; "cycloalkyl" is intended to include saturated ring groups, such as cyclopropyl, cyclobutyl, cyclopentyl, cyclohexyl, cycloheptyl and cyclooctyl; and "biycloalkyl" is intended to include saturated bicyclic ring groups such as [3.3.0]bicyclooctane, [4.3.0]bicyclononane, [4.4.0]bicyclodecane (decalin) and [2.2.2]bicyclooctane. "Alkenyl" is intended to include hydrocarbon chains of either a straight or branched configuration and one or more unsaturated carbon-carbon bonds which may occur in any stable point along the chain, such as ethenyl and propenyl; and "alkynyl" is intended to include hydrocarbon chains of either a straight or branched configuration and one or more triple carbon-carbon bonds which may occur in any stable point along the chain, such as ethynyl and propynyl. "Halo" as used herein refers to fluoro, chloro, bromo, and iodo; and "counterion" is used to represent a small, negatively charged species such as chloride, bromide, hydroxide, acetate, sulfate and sulfate.

As used herein, "aryl" or "aromatic residue" is intended to mean phenyl or naphthyl; "carbocyclic" is intended to mean any stable 5- to 7- membered monocyclic or bicyclic or 7- to 14-membered bicyclic or tricyclic carbon ring, any of which may be saturated, partially unsaturated, or aromatic, for example, indanyl or tetrahydronaphthyl (tetralin).

As used herein, the term "heterocycle" is intended to mean a stable 5- to 7- membered monocyclic or bicyclic or 7- to 10-membered bicyclic heterocyclic ring which is either saturated or unsaturated, and which consists of carbon atoms and from 1 to 3 heteroatoms selected from N, O and S and wherein the nitrogen and sulfur heteroatoms may optionally be oxidized, and the nitrogen may optionally be quaternized, and including any bicyclic group in which any of the above-defined heterocyclic rings is fused to a benzene ring. The heterocyclic ring may be attached to its pendant group at any heteroatom or carbon atom which results in a stable structure. The heterocyclic rings described herein may be substituted on carbon or on a nitrogen atom if the resulting compound is stable. Examples of such heterocycles include pyridyl, pyrimidinyl, furanyl, thienyl, pyrrolyl, pyrazolyl, imidazolyl, tetrazolyl, benzofuranyl, benzothiophenyl, indolyl, indolenyl, quinolinyl, isoquinolinyl or benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl and octahydroisoquinolinyl. The term "substituted", as used herein, means that an one or more hydrogen on the designated atom is replaced with a selection from the indicated group, provided that the designated atom's normal valency is not exceeded, and that the substitution results in a stable compound.

By "stable compound" or "stable structure" is meant herein a compound that is sufficiently robust to survive isolation to a useful degree of purity from a reaction mixture, and formulation into an efficacious therapeutic agent.

As used herein, "pharmaceutically acceptable salts and prodrugs" refer to derivatives of the disclosed compounds that are modified by making acid or base salts, or by modifying functional groups present in the compounds in such a way that the modifications are cleaved, either in routine manipulation or in vivo, to the parent compounds. Examples include mineral or organic acid salts of basic residues such as amines; alkali or organic salts of acidic residues such as carboxylic acids; and acetate, formate and benzoate derivatives of alcohols and amines.

Pharmaceutically acceptable salts of the compounds of the invention can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

### Synthesis

The compounds of the present invention may be synthesized using the general synthetic procedures described below.

Compounds of the invention wherein:
W is -N(R²²)C(=Z)N(R²³)-
can be synthesized from diamines of formula (V) : 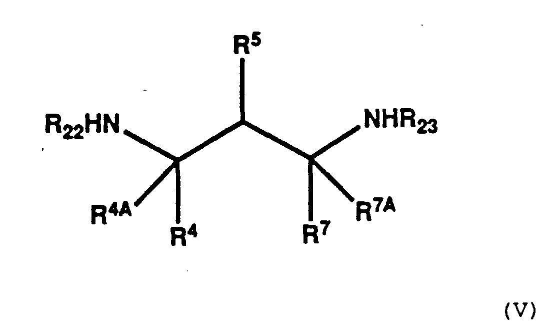 which can in turn be synthesized as described in EP-A-0 402 646.

The compounds of formula (V) can be cyclized to form compounds of formula (VI) under conditions normally used to form cyclic ureas, as is known to one skilled in the art. Reagents J-(C=Z)-J', where J and J' are leaving groups, are employed, preferably under relatively dilute conditions (for example, less than about 0.1 M), to effect ring closure to provide compounds of formula (I). Many examples of J and J' are known; preferred are carbonyl diimidazole, thiocarbonyldiimidazole, phosgene, thiophosgene, diphenyl carbonate, or diphenyl thiocarbonate.

For compounds of the invention where R²⁰ is -OH, it is advantageous to protect the free hydroxyl before cyclization. Protecting groups used can include any of those listed in Greene, Protective Groups in Organic Synthesis, Chapter 2, Wiley, NY (1981). The preferred protecting groups are trimethylsilylethoxymethyl (SEM), methoxyethoxymethyl (MEM), or methoxymethyl (MOM).

Cyclization of compounds of formula (V) results in structure (VI) (i.e., structure (I) wherein W is -N(R²²)C(=O) N(R²³)-).

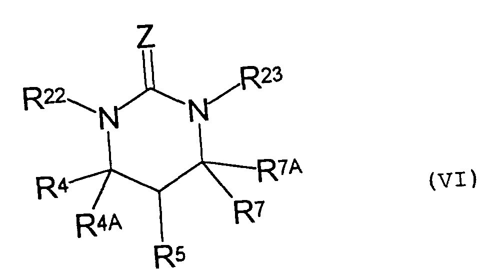

Another, preferred method to form compounds of formula (VI), in cases wherein R²² and R²³ are linked to their respective nitrogens by a CH₂ residue, is to cyclize a compound of structure (V) where R²² and R²³ are hydrogen, and to alkylate the nitrogens using a base, a phase transfer catalyst, and an alkylating agent, using methods well known in the art. The preferred base is sodium hydride, and the preferred alkylating agents are R²²Y and R²³Y, wherein Y is a halogen, triflate, or mesylate, preferably a bromide or iodide. Preferred conditions are in polar aprotic solvents between 0 and 100 °C.

Cleavage of protecting groups, if employed, yields structures of formula (I) wherein R⁵ is hydroxyl.

When R⁵ is other than OR²⁰, some chemical manipulation of functional groups may need to be performed in the preparation of the compounds of formula (V) or (VI) as is appreciated by one of skill in the art of organic synthesis.

Compounds of structure (I) described above wherein Z = O can be converted to the thio derivatives, Z = S, using standard conditions (March, Advanced Organic Chemistry, p. 792, Wiley, NY, 1985); preferred is the use of the disulfide described in Bull. Soc. Chim. Belges 1978, 223.

Structures (I) described above wherein Z = O can be converted to the imino derivatives, Z = NR²⁴, using standard conditions. When R²⁴ is OH or O-alkyl, the oximes can be formed and alkylated if desired as described in March, Advanced Organic Chemistry, pp. 359, 805, Wiley, NY, 1985. The hydrazones and imines can be formed similarly (ibid, pp. 533, 797).

Compounds wherein W is -N(R²²)C(=O)N(R²³)-can likewise be synthesized as shown in the scheme shown below. If necessary, intermediates described herein can be manipulated by methods known to those skilled in the art of organic synthesis to provide compounds within the scope of the invention.

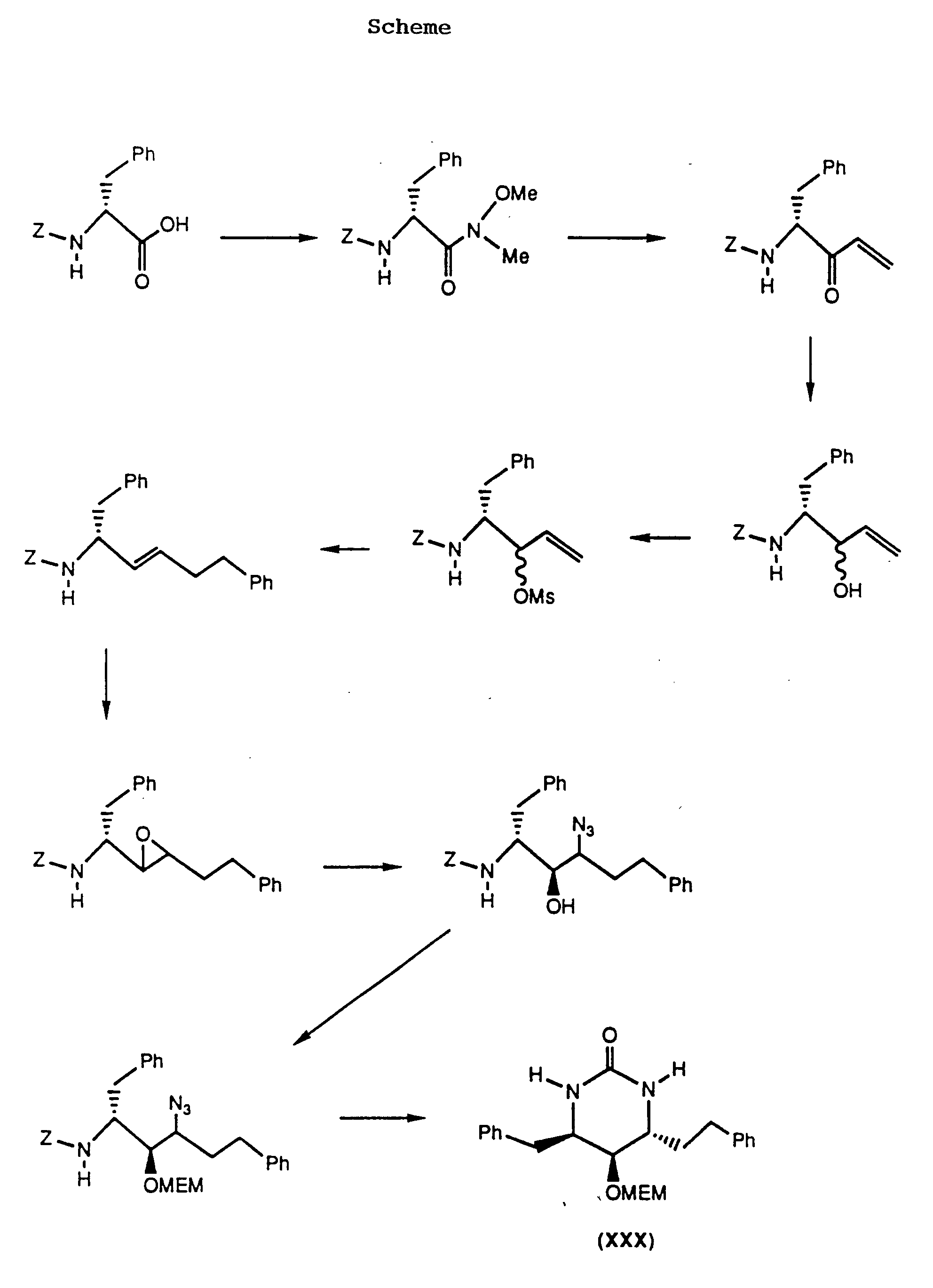

The synthesis of compounds of the invention is described in further detail below.

### Procedure 1

General alkylation/hydrolisis procedure:

1 mmol of the starting compound dissolved in dry DMF (5 ml) was added to a flask containing sodium hydride (10 mmol, that had been washed with hexane, 3x20 ml) in DMF (5 ml). The solution was stirred at room temperature under nitrogen for 5 min. Evolution of hydrogen gas was observed. The appropriate alkyl bromide (5 mmol) was added and the solution was stirred at room temperature under nitrogen for 1h. Hindered alkyl bromides required heating at 40-70 °C for up to 5 h. TLC (40/60 ethyl acetate/hexane) was used to ensure that no starting material remained. The solution was quenched with methanol (1 ml), partitioned between ether (60 ml) and water (50 ml) and the organic layer was removed. The aqueous layer was washed with ether (50 ml), the organic layers combined and washed with water (4x30 ml), brine (30 ml), dried over MgSO₄, filtered and evaporated. In cases where the alkyl bromide contained nitrogen, 1 N NaOH was used in place of water.

The crude product was hydrolyzed directly in methanol (10 ml) and 4 N HCl/dioxane (5 ml) for up to 16 h at room temperature. The solution was evaporated and chromatographed directly on SiO₂ to afford the bis-alkylated cyclic ureas. Where nitrogen was present, the solutions were first basified with 1 N NaOH and extracted with ethyl acetate, dried over MgSO₄, filtered, evaporated and chromatographed.

The structures of the Examples below are sown in Tables 1a and 1b.

### Example 1X (Reference Example)

The physical data of 1X (structure shown in Table 1a) are as follows: Example 1X: mp 210-212 °C. MS: 435(M+1,100). NMR(CDCl₃)δ 7.18-7.35(m, 10H, Ph), 4.06(s, 2H, CHOH), 3.68(br d, 2H, NCH), 3.55(q, 2H, NCHCH), 3.1(m, 4H, PhCH₂), 2.55(s, 2H, OH), 2.05(q, 2H, CHCH), .9(m, 2H, NCH₂CH), .42(m, 4H, -CH₂-), .008(m, 4H, -CH₂-).

### Example 7V

A solution of Ex. 1X (112 mg, 0.26 mmol) in methylene chloride (5 ml) was cooled in an ice bath at 0 °C and treated with DAST (110 mg, 1.1 mmol). The mixture was stirred for 10 minutes and then washed with sat'd NaHCO₃(aq), brine, and dried over MgSO₄. The solution is filtered and the solvent removed on a rotary evaporator and the resulting residue is HPLC chromatographed on silica gel (65% EtOAc/Hexanes) to give 40 mg of a colorless residue. HRMS calculated for C₂₇H₃₃N₂O₂F: 437.2604; found: 437.2593.

### Example 7o (Reference Example)

A solution of Ex. 3U (600 mg, 1.9 mmol) in pyridine (10 ml) is treated with methanesulfonyl chloride (170 mg, 1.5 mmol) and stirred at room temperature for 3 hours. The mixture is quenched with 5 ml of methanol. The solution is concentrated on a rotary evaporator and the resulting residue is dissolved in ethyl acetate and then washed with dilute HCl(aq), brine, and dried over MgSO4. The solution is filtered and the solvent removed on a rotary evaporator and the resulting residue is chromatographed on silica gel (40% EtOAc/Hexanes) to give Example 7o (420 mg ) as a white solid. ms (M+H)⁺ = 685.

### Example 7Y

A solution of Example 7o (100 mg, 0.15 mmol) in DMF (4 ml) was treated with NaN₃ (100 mg, 1.5 mmol) and heated at 80 °C for 2 hrs and then stirred overnight at 40 °C. The solution was diluted with water and the resulting white solid is extracted into ethyl acetate. The organic extract is washed with water, brine, and dried over MgSO₄. The solution is filtered and the solvent removed on a rotary evaporator and the resulting residue is HPLC chromatographed on silica gel (50% EtOAc/Hexanes) to give Example 7Y (80 mg ) as a white solid. MS (M+H)⁺ = 632. IR (CHCl₃) 2214 cm⁻¹ for N₃.

### Example 7W

A solution of example 1C (160 mg, 0.39 mmol) in methylene chloride (5 ml) was cooled in an ice bath at 0 °C and treated with DAST (63 mg, 0.4 mmol). The mixture was stirred for 10 minutes and then washed with sat'd NaHCO₃(aq), brine, and dried over MgSO₄. The solution is filtered and the solvent removed on a rotary evaporator and the resulting residue is HPLC chromatographed on silica gel (50% EtOAc/Hex) to give 80 mg of a colorless residue. HRMS calculated for C₂₅H₃₀N₂O₂F: 409.2291; found: 409.2291.

### Example 7X

A solution of example 3U (100 mg, 0.16 mmol) in methylene chloride (5 ml) was cooled in an ice bath at 0 °C and treated with DAST (26 mg, 0.16 mmol). The mixture was stirred for 10 minutes and then washed with sat'd NaHCO₃(aq), brine, and dried over MgSO₄. The solution is filtered and the solvent removed on a rotary evaporator and the resulting residue is HPLC chromatographed on silica gel (50% EtOAc/Hex) to give 35 mg of a white foam. HRMS calculated for C₄₁H₃₈N₂O₂F: 609.2917; found: 609.2911.

### Example 8A

A solution of example 1X (2.06 g, 4.74 mmol) in methylene chloride was treated with diisopropylethylamine (1.53 g, 11.8 mmol), MEM-Cl (0.71 g, 5.7 mmol) and heated to reflux for 5 hours and let stir overnight at rt. The solution is concentrated on a rotary evaporator the residue is HPLC chromatographed on silica gel (5 % MeOH/CHCl₃) to give 1.3 g of the mono-MEM mono-ol intermediate. MS: (CI, NH₃) (M+H)⁺ = 523.4.

A solution of mono-MEM mono-ol intermediate from above (1.0 g, 1.9 mmol) in THF was treated with triphenylphosphine (1.0 g, 3.8 mmol), diethylazadicarboxylate (DEAD) (0.7 g, 4.0 mmol), and chloroacetic acid (0.4 g, 4.2 mmol). The solution is stirred overnight at rt. The solvent is evaporated and the resulting residue is chromatographed on silica gel (50% EtOAc/Hex) to give 0.9 g of the chloroacetate intermediate. MS: (CI, NH₃) (M+H)⁺ = 599.3 (100%); 600 (39%).

The chloroacetate intermediate (0.9 g, 1.5 mmol) was dissolved in MeOH (15 ml) and treated with NaOH(aq) (4 ml, 1N) and stirred at rt for 15 min. The solution was evaporated to dryness and the residue partitioned between water and ethyl acetate. The organic layer was washed with water and brine and then dried over MgSO₄. The solution is the filtered, concentrated and the residue is HPLC chromatographed on silica gel (85% EtOAc/Hex) to give 400 mg of example 8A. MS: (CI, NH₃) (M+H)⁺ = 523.4.

### Example 7Z

A solution of example 8A (100 mg, 0.2 mmol) in MeOH is cooled in an ice bath and treated with HCl _{(g)} for 20 min and then stirred for an additional 40 min at 0°C. The solution is then evaporated to dryness at rt and the residue is HPLC chromatographed on silica gel (80% EtOAc/Hex) to give 48 mg of example 7Z as a white foam. MS: (CI, NH₃) (M+H)⁺ = 435.2

### Example 8C

A solution of example 8A (160 mg, 0.3 mmol) in methylene chloride (10 ml) is cooled to 0°C in an ice bath and treated with DAST (50 mg, 0.3 mmol). The solution is stirred at rt for 15 min and then quenched with water. The organic layer is washed with water and brine and dried over MgSO₄. The solution is filtered, the solvent evaporated and the residue HPLC chromatographed on silica gel (50% EtOAc/Hex) to give 100 mg of example 8C. MS: (CI, NH₃) (M+H)⁺ = 525.4

### Example 8B

A solution of example 8C (70 mg, 0.13 mmol) in MeOH is cooled in an ice bath and treated with HCl (g) for 20 min and then stirred for an additional 40 min at 0°C. The solution is then evaporated to dryness at rt and the residue is HPLC chromatographed on silica gel (80% EtOAc/Hex) to give 40 mg of example 8B as a white foam. MS: (CI, NH₃) (M+H)⁺ = 437.3

### Example 8E

A solution of Example 5F (500 mg, 0.7 mmol) in methylene chloride (10 ml) is cooled to 0°C in an ice bath and treated with DAST (112 mg, 0.7 mmol). The solution is stirred at 0°C for 15 min and then quenched with sat'd NaHCO₃. The organic layer is washed with water and brine and dried over MgSO₄. The solution is filtered, the solvent evaporated and the residue HPLC chromatographed on silica gel (50% EtOAc/Hex) to give 250 mg of example 8E as a white foam. MS: (CI, NH₃) (M+H)⁺ = 721

### Example 8F

A solution of example 8E (200 mg, 0.28 mmol) in MeOH is cooled in an ice bath and treated with gaseous HCl for 20 min and then stirred for an additional 40 min at 0°C. The solution is then evaporated to dryness at rt and the residue is HPLC chromatographed on silica gel (5% MeOH/CHCl₃) to give 120 mg of example 8F as a white foam. MS: (CI, NH₃) (M+H)⁺ = 541

### Example 8G (via Alkene Intermediate XXIX):

A solution of Example 7O (150 mg, 0.22 mmol) in DMF was treated with sodium iodide (160 mg, 1.1 mmol) and heated at 90°C for 2 h. The mixture is cooled to room temperature, diluted with water and the precipitate is extracted into CH₂Cl₂. The extract is washed with water and brine, dried over MgSO₄ and evaporated to give a yellow oil. This is HPLC chromatographed on silica gel (50% EtOAc/Hex) to give 50 mg of alkene intermediate (XXIX) as a white solid. MS: (CI, NH₃) (M+H)⁺ = 589 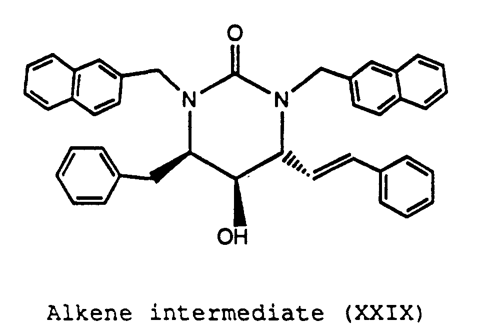 A solution of alkene intermediate (XXIX) (40 mg, 0.07 mmol) in THF was treated with 20 mg of 10% Pd/C and hydrogenated in a Parr Hydrogenator at 3.4 bar (50 psi) overnight. The catalyst was filtered off and the filtrate concentrated. The resulting residue was HPLC chromatographed on silica gel (70% EtOAc/Hex) to give 10 mg of Example 8G as a white solid. MS: (CI, NH₃) (M+H)⁺ = 591.5

### Example 8H

### Method 1.

A. Synthesis of 6-membered ring cyclic urea (XXX):

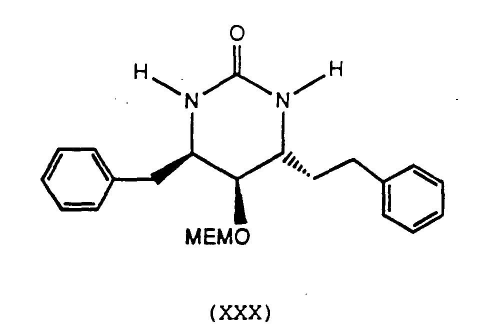

The synthesis of the six-membered cyclic urea (XXX) is outlined in the Scheme shown above. A solution of N-Cbz-D-phenylalanine N,O-dimethylhydroxylamide (33.5 g , 0.098 mol) in ether was cooled to 0°C and treated with 300 ml of a 1 M solution of vinyl magnesium bromide in THF. The mixture was stirred for 30 min and then poured into an ice cold solution of 1 N HCl (500 ml). The mixture was extracted into ether and the extracts washed with water and brine. The organic layer was dried over MgSO₄, filtered and concentrated to give the desired vinyl ketone as a thick, light yellow residue which was used without further purification. MS: (CI, NH₃) (M+H)⁺ = 310 (77%); (M+NH₄)⁺ = 327.1 (100%).

The crude ketone was dissolved in methanol (350 ml) and treated with cerium trichloride heptahydrate (37.2 g, 0.1 mol) and cooled in an ice bath. While stirring vigorously sodium borohydride (3.78 g 0.1 mol) was added slowly, a small portion at a time, over a period of 30 min. After the addition was complete the mixture was allowed to warm to room temperature and stirred for an additional 1 h. The solvent was removed under vacuum on a rotorary evaporator and the residue was partitioned between 1 N HCl and methylene chloride. The organic layer was washed with water, brine, and then dried over MgSO₄, filtered and concentrated to give the desired allylic alcohol as an off-white solid which was used without further purification.

A solution of the crude allylic alcohol and diisopropylethylamine (30 g, 0.23 mol) in methylene chloride was cooled in an ice bath and treated dropwise with methanesulfonyl chloride (28 g, 0.24 mol). The solution was stirred for 30 min, then washed sequentially with 1 N HCl, water, brine and dried over MgSO₄. The solution was filtered and concentrated to give the crude mesylate as a thick oil. To a flamed-dried flask was added copper cyanide (12 g, 0.144 mol) and 100 ml of THF. The flask was cooled to -78 °C under nitrogen atmosphere. A solution of benzylmagnesium chloride (360 ml, 2M in THF, 0.72 mol) was added via syringe and the resulting thick solution was stirred at -60°C for 20 min and at 0 °C for 30 min. The solution was then cooled to - 78°C and a solution of the mesylate in 130 ml of THF was added via syringe. The solution was stirred at -60°C for 45 min and then poured into a mixture of 1 N HCl/ice. This was extracted into ethyl acetate and the organic layer was washed sequentially with NH₄Cl (aq), NH₄OH, brine, dried over MgSO₄, filtered and concentrated. The resulting residue is chromatographed on silica gel (hexane, then 10% EtOAc/Hex) to give 11.7 g of the desire alkene as a white solid. MS: (CI, NH₃) (M+H)⁺ = 386.3 (98%); (M+NH₄)⁺ = 403.2 (100%).

A solution of the above alkene (11.0 g, 0.029 mol) in methylene chloride (75 ml) was cooled to 0°C in an ice bath and treated with 60% m-chloroperbenzoic acid (14.0 g, 0.049 mol). The solution was stirred 0°C for 7 h until TLC analysis showed no starting material remained. A precipitate formed during this time. The suspension was diluted with methylene chloride and washed sequentially with 1 N Na₂S₂O₃, 1 N sodium hydroxide, water, brine, dried over MgSO₄, filtered and concentrated to give the epoxide as a thick oil which was used without further purification.

To solution of crude epoxide in 80 ml of DMF was added sodium azide (20 g , 0.3 mol), ammonium chloride (2.5 g , 0.047 mol) and 20 ml of water. The mixture was heated at 90 °C for 3 h and then stirred at rt overnight. The solvent was removed under high vacuum on a rotorary evaporator and the residue was partitioned between water and methylene chloride. the organic layer was washed with water and brine, dried over MgSO₄, filtered and concentrated to give a residue. This was then chromatographed on silica gel (20% EtOAc/Hex) to give 7.4 g of the azide alcohol as a white solid. MS: (CI, NH₃) (M+H)⁺ = 445.0 (25%); (M+NH₄-BnOH)⁺ = 462.2 (100%).

A solution of the azide alcohol above (7.2 g, 0.016 mol) in methylene chloride was treated with diisopropylethylamine (4.2 g , 0.032 mol) and MEM-Cl (4.0 g 0.032 mol) and heated to reflux overnight (18 h ). The mixture was concentrated and the residue chromatographed on silica gel (20% EtOAc/Hex - 35% EtOAc/Hex) to give 7.7 g of the MEM protected azido alcohol as a colorless oil. MS: (CI, NH₃) (M+H)⁺ = 533.2 (100%).

To a solution of MEM protected azido alcohol (5.7 g 0.0107 mol) in ethyl acetate was added 2 ml of acetic acid and 1 g of Pearlman's catalyst (10 % Pd(OH)₂ on Carbon) and the solution was hydrogenated at 3.8 bar (55psi) for 22 h. The solution was filtered through Celite and the filtrate was extracted with 1 N HCl (organic layer turn orange). The acidic aqueous extract was made basic with 50% NaOH (while cooling in an ice bath) and the precipitate is extracted into ethyl acetate. The organic layer is washed with water, brine, dried over MgSO₄, filtered and concentrated to give 2.5 g of the MEM protected diamino alcohol as a colorless oil. MS: (CI, NH₃) (M+H)⁺ = 373.1 (100%).

To a solution of the MEM protected diamino alcohol (2.5 g 0.0067 mol) in THF was added 1,1-carbonyldiimidazole (1.1 g, 0.0067 mol) and stirred over night at room temperature. The solution was concentrated and the residue partitioned between 1 N HCl and CH₂Cl₂. The organic layer is washed with brine, dried over MgSO₄, filtered and concentrated. The residue is HPLC chromatographed on silica gel ( 5% MeOH/CHCl₃) to give 1.2 g of the MEM protected 6-membered ring cyclic urea (XXX) as a white solid. MS: (CI, NH₃) (M+H)⁺ = 399.1 (100%).

B. The MEM protected 6-membered ring cyclic urea (XXX) (100 mg, 0.27 mmol) was alkylated with cyclopropylmethylbromide (250 mg , 1.8 mmol) followed by removal of the MEM group, as described in general procedure 1, to give after chromatography on HPLC (silica gel, 10% MeOH/CHCl₃) 20 mg of Example 8H as a clear, viscous residue. MS: (CI, NH₃) (M+H)⁺ = 419.4 (100%).

### Method 2.

A solution of Example 8A (160 mg, 0.3 mmol) and thiocarbonyldiimidazole (55 mg, 0.3 mmol) in THF was heated to reflux for 4 h. The mixture was evaporated and the residue chromatographed on silica gel (50% EtOAc/Hex) to give 34 mg (0.055 mmol) of the corresponding thiocarbamate. The thiocarbamate was dissolved in 2 m¹ of toluene and heated to reflux. To the refluxing solution was added tributyltin hydride (32 mg, 0.1 mmol) and 2 mg of AIBN. The mixture was refluxed for 1 hour, concentrated, and the residue chromatography on HPLC (silica gel, 65% EtOAc/Hex) to give 20 mg of clear colorless oil. The oil was dissolved in MeOH, cooled in an ice bath and gaseous HCl was bubbled through the solution for 30 mins. The solution was then stirred at room temperature overnight, concentrated and the residue chromatography on HPLC (silica gel, 10% MeOH/CHCl₃) to give 10 mg of Example 8H as a clear, viscous residue. MS: (CI, NH₃) (M+H)⁺ = 419.2 (100%).

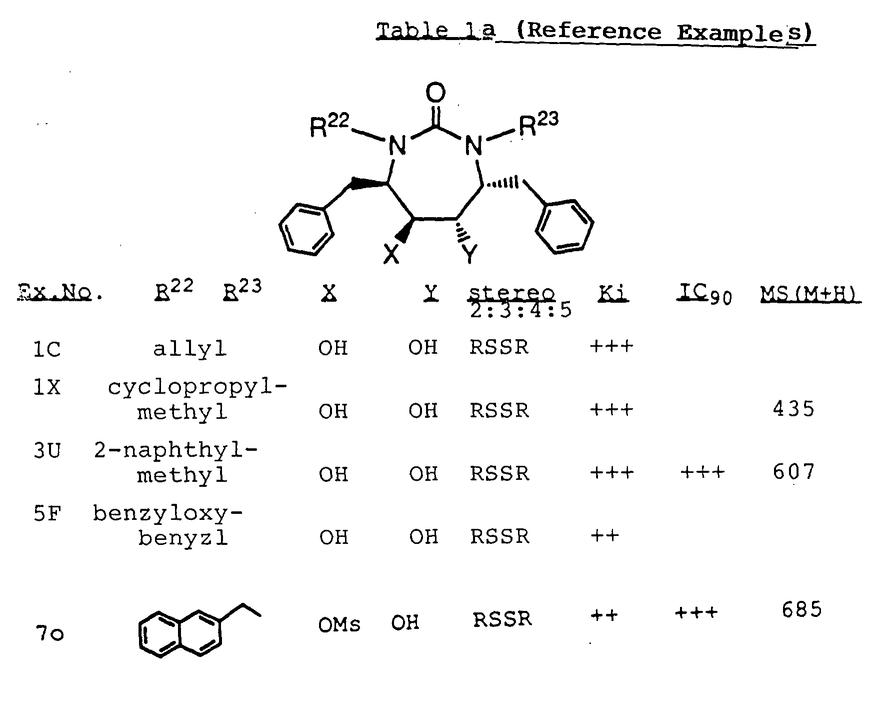

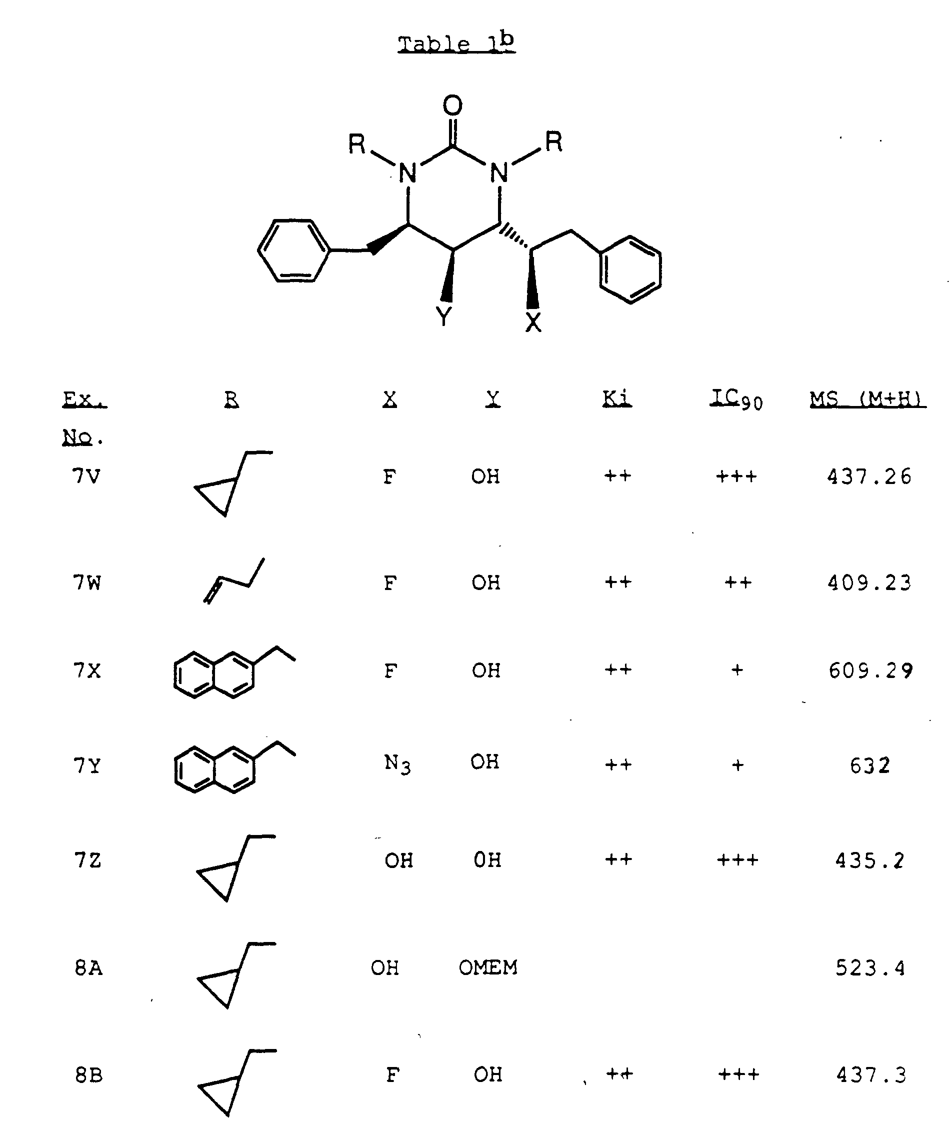

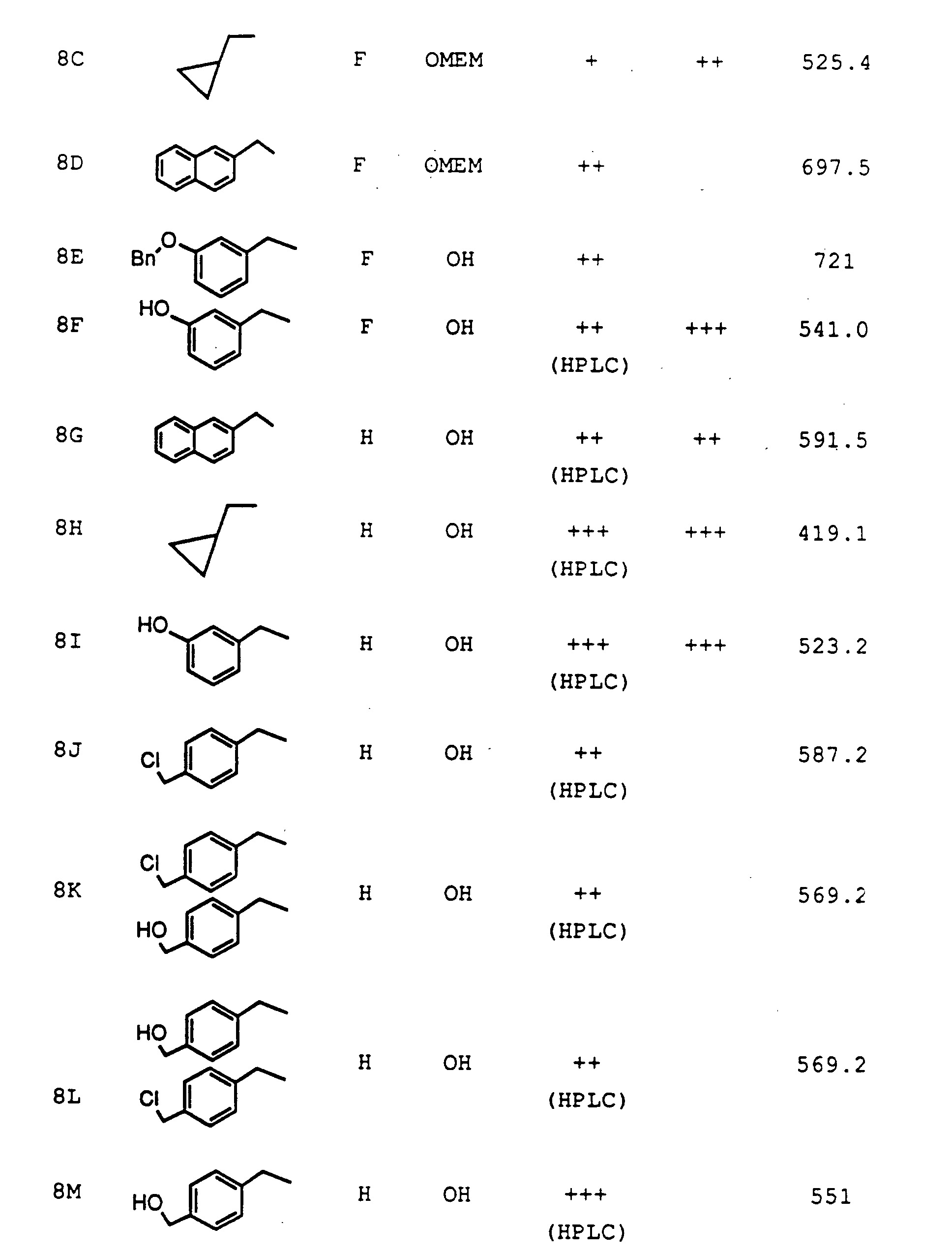

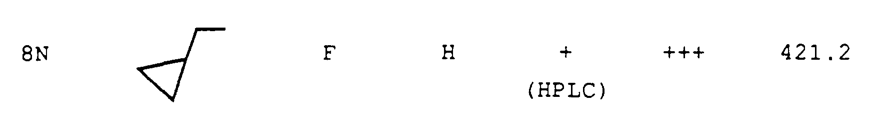

Using the above-described techniques or variations thereon appreciated by those of skill in the art of chemical synthesis, the compounds of Tables 2 and 3 (shown below) can also be prepared.

### Utility

The compounds of formula (I) possess retroviral protease inhibitory activity and are therefore useful as antiviral agents for the treatment of viral diseases. More particularly, the compounds of formula (I) possess HIV protease inhibitory activity and are effective as inhibitors of HIV growth. The protease inhibitory activity of the compounds of the present invention is demonstrated using standard assays of protease activity, for example, using the assay described below for assaying inhibitors of HIV protease activity. The ability of the compounds of the present invention to inhibit viral growth or infectivity is demonstrated in standard assay of viral growth or infectivity, for example, using the assays described below.

A compound is considered to be active if it has an IC₅₀ or Ki value of less than 1 mM for the inhibition of HIV protease or HIV viral growth or infectivity.

### HIV Protease Inhibition Assay- Spectroscopic Method

### Materials:

Protease: Inclusion bodies of E. coli harboring plasmid containing HIV protease under the control of an inducible T7 promoter were prepared according to Cheng et. al (1990) Gene 87: 243. Inclusion bodies were solubilized in 8 M urea, 50 mM tris pH 8.0. Protease activity was recovered by dilution 20-fold into buffer containing 50 mM sodium acetate pH 5.5, 1 mM EDTA, 10% glycerol and 5% ethylene glycol. Enzyme was used at a final concentration of 1.0-10 µg/ml.

Substrate: Peptide of sequence: Ala-Thr-His-Gln-Val-Tyr-Phe(NO₂)-Val-Arg-Lys-Ala, containing ρ-nitrophenylalanine (Phe(NO₂)), was prepared by solid phase peptide synthesis as previously described by Cheng et al. (1990) Proc. Natl. Acad. Sci. USA 87: 9660. Stock solutions of 10 mM were prepared in DMSO.

Inhibitory compounds were dissolved in sufficient DMSO to make 2.5 or 25 mM stock solutions. All further dilutions were done in DMSO.

### Reactions:

Compound (1-5 µl) and HIV protease were mixed in buffer containing 50 mM MES, pH 6.5, 1 M NaCl, 1 mM EDTA, 1 mM dithiothreitol, 10% glycerol. Reactions were initiated by the addition of peptide substrate to a final concentration of 240 µM, and absorbance at 300 nM monitored for 10 min. Values of Ki for inhibitor binding were determined from percent activity measurements in the presence and absence of known concentration of inhibitor, using a value of 0.07 mM for the Km of the substrate (Cheng et al. (1990) Proc. Natl. Acad. Sci. USA 87: 9660).

The HIV-1 protease inhibitory activity of representative compounds of the invention is shown in Table 1b.

### HIV Protease Inhibition Assay- HPLC Method

### Materials:

Protease: Inclusion bodies of E. coli harboring plasmid containing plasmid T1718R with a synthetic gene coding for a single-chain tethered dimer of HIV protease were prepared as described in Cheng et al. (Proc. Natl. Acad. Sci. USA, 87, 9660-9664, 1990). Active protease was prepared as described therein by extraction with 67% acetic acid, dilution 33-fold with water, dialysis against water and then against a "refolding buffer" consisting of 20 mM MES, 1 mM dithiothreitol and 10% glycerol. Protease was stored as a stock preparation at 10 µM in refolding buffer.

Substrate: Peptide of sequence: aminobenzoyl-Ala-Thr-His-Gln-Val-Tyr-Phe(NO₂)-Val-Arg-Lys-Ala containing p-nitrophenylalanine, was prepared by solid phase synthesis as previously described Cheng et al., op.cit. Stock solutions of 2 mM substrate were prepared in DMSO.

Inhibitory compounds were dissolved in sufficient DMSO to make 3 mM stock solutions. All further dilutions were prepared in "assay buffer": 1 M NaCl, 50 mM MES, pH 5.5, 1 mM EDTA, 1mM DTT, 20% glycerol.

### Reactions:

Enzyme reaction: In a 2 ml screw-cap centrifuge tube were added 50ul protease (final concentration 0.25 nM) and 0.1 ml inhibitory compound (final concentration 0.1-12,500). After 15 min preincubation at room temperature, the reaction was started with the addition of 0.05 ml substrate (final concentration 5 µM). Incubation was carried out at 30°C for 1 h. The reaction was stopped with 1 ml 0.1 M ammonium hydroxide.

HPLC measurement of product formation: The product (aminobenzoyl-Ala-Thr-His-Gln-Val-Tyr) was separated from substrate on a Pharmacia MonoQ anion exchange column. The injection volume was 0.2 ml. The mobile phases were: A (20 mM trisHCl, pH 9.0, 0.02% sodium Azide, 10% acetonitrile), B (20 mM tris HCl, pH 9.0, 0.02% sodium azide, 0.5 M ammonium formate, 10% acetonitrile). The mobile phases were pumped at 1 ml/min, with a gradient from 0 to 30% B in 5 min, 100 % B for 4 min to wash the column, and a re-equilibration for 4 min. The retention time of the product was 3.6 min. Detection with a Shimadzu model RF535 fluorescence monitor was at 330 nm (excitation) and 430 (emission). The Ki was calculated from the formula Ki = I/(((Km+S-FA*S)/(FA*Km))-1) where I = inhibitory concentration, S = substrate concentration, FA = fractional activity = cm peak height with inhibitor/cm peak height without inhibitor, and Km = Michaelis constant = 20 µM.

### HIV Yield Reduction Cell Assay

Materials: MT-2, a human T-cell line, was cultured in RPMI medium supplemented with 5% (v/v) heat inactivated fetal calf serum (FCS), L-glutamine and gentamycin. Human immunodeficiency virus strains, HIV (3B) and HIV(RF) were propagated in H-9 cells in RPMI with 5% FCS. Poly-L-lysine (Sigma) coated cell culture plates were prepared according to the method of Harada et al. (1985) Science 229: 563-566. MTT, 3-(4,5-dimethyl-thiazol-2yl)-2,5-diphenyltetrazolium bromide, was obtained from Sigma.

Method: Test compounds were dissolved in dimethylsulfoxide to 5 mg/ml and serially diluted into RPMI medium to ten times the desired final concentration. MT-2 cells (5 x 10⁵/ml) in 2.3 ml were mixed with 0.3 ml of the appropriate test compound solution and allowed to sit for 30 minutes at room temperature. HIV (3B) or HIV (RF) (∼5 x 10⁵ plaque forming units/ml) in 0.375 ml was added to the cell and compound mixtures and incubated for one hour at 36°C. The mixtures were centrifuged at 1000 rpm for 10 minutes and the supernatants containing unattached virus were discarded. The cell pellets were suspended in fresh RPMI containing the appropriate concentrations of test compound and placed in a 36°C, 4% CO₂ incubator. Virus was allowed to replicate for 3 days. Cultures were centrifuged for 10 minutes at 1000 rpm and the supernatants containing cell free progeny virus were removed for plaque assay.

The virus titers of the progeny virus produced in the presence or absence of test compounds were determined by plaque assay. Progeny virus suspensions were serially diluted in RPMI and 1.0 ml of each dilution was added to 9 ml of MT-2 cells in RPMI. Cells and virus were incubated for 3 hours at 36°C to allow for efficient attachment of the virus to cells. Each virus and cell mixture was aliquoted equally to two wells of a six well poly-L-lysine coated culture plate and incubated overnight at 36°C, 4% CO₂. Liquid and unattached cells were removed prior to the addition of 1.5 mL of RPMI with 0.75% (w/v) Seaplaque agarose (FMC Corp.) and 5% FCS. Plates were incubated for 3 days and a second RPMI/agarose overlay was added. After an additional 3 days at 36°C, 4% CO₂, a final overlay of phosphate-buffered saline with 0.75% Seaplaque agarose and 1 mg MTT/ml was added. The plates were incubated overnight at 36°C. Clear plaques on a purple background were counted and the number of plaque forming units of virus was calculated for each sample. The antiviral activity of test compounds was determined by the percent reduction in the virus titer with respect to virus grown in the absence of any inhibitors.

### HIV Low Multiplicity Assay

Materials: MT-2, a human T-cell line, was cultured in RPMI medium supplemented with 5% (v/v) heat inactivated fetal calf serum (FCS), L-glutamine and gentamycin (GIBCO). Human immunodeficiency virus strains HIV (3B) and HIV (RF) were propagated in H-9 cells in RPMI with 5% FCS. XTT, benzene-sulfonic acid, 3,3'-[1-[(phenyl-amino)carbonyl]-3,4-tetrazolium]bis(4-methoxy-6-nitro)-, sodium salt, was obtained from Starks Associates, Inc.

Method: Test compounds were dissolved in dimethylsulfoxide to 5 mg/ml and serially diluted into RPMI medium to ten times the desired final concentration. MT-2 cells (5 x 10⁴/0.1 ml) were added to each well of a 96 well culture plate and 0.02 ml of the appropriate test compound solution was added to the cells such that each compound concentration was present in two wells. The cells and compounds were allowed to sit for 30 minutes at room temperature. HIV(3B) or HIV(RF) (∼5 x 10⁵ plaque forming units/ml) was diluted in medium and added to the cell and compound mixtures to give a multiplicity of infection of 0.01 plaque forming unit/cell. The mixtures were incubated for 7 days at 36°C, during which time the virus replicated and caused the death of unprotected cells. The percentage of cells protected from virus induced cell death was determined by the degree of metabolism of the tetrazolium dye, XTT. In living cells, XTT was metabolized to a colored formazan product which was quantitated spectrophotometrically at 450 nm. The amount of colored formazan was proportional to the number of cells protected from virus by the test compound. The concentration of compound protecting either 50% (IC₅₀) or 90% (IC₉₀) with respect to an uninfected cell culture was determined.

In the Tables herein the Ki values were determined using the assay conditions described above under HIV Protease Inhibtion Assay. The Ki values are indicated as follows: +++ = <10 nM; ++ = 10 nM to 1 µM; + = >1 µM.

In the Tables herein the IC₉₀ values were determined using the assay conditions described above under HIV Low Multiplicity Assay. The IC₉₀ values are indicated as follows: +++ = <10 µg/ml; ++ = 10 to 100 µg/ml; + = >100 µg/ml.

### Dosage and Formulation

The antiviral compounds of this invention can be administered as treatment for viral infections by any means that produces contact of the active agent with the agent's site of action, the viral protease, in the body of a mammal. They can be administered by any conventional means available for use in conjunction with pharmaceuticals, either as individual therapeutic agents or in a combination of therapeutic agents. They can be administered alone, but generally administered with a pharmaceutical carrier selected on the basis of the chosen route of administration and standard pharmaceutical practice.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be . 0.001 to 1000 milligrams per kilogram of body weight, with the preferred dose being 0.1 to 30 mg/kg.

Dosage forms (compositions suitable for administration contain from 1 milligram to 100 milligrams of active ingredient per unit. In these pharmaceutical compositions the active ingredient will ordinarily be present in an amount of 0.5-95% by weight based on the total weight of the composition.

The active ingredient can be administered orally in solid dosage forms, such as capsules, tablets, and powders, or in liquid dosage forms, such as elixirs, syrups, and suspensions. It can also be administered parenterally, in sterile liquid dosage forms.

Gelatin capsules contain the active ingredient and powdered carriers, such as lactose, starch,cellulose derivatives, magnesium stearate and stearic acid.

Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propyl-paraben, and chlorobutanol.

Suitable pharmaceutical carriers are described in Remington's Pharmaceutical Sciences, Mack Publishing Company, a standard reference text in this field.

Useful pharmaceutical dosage-forms for administration of the compounds of this invention can be illustrated as follows:

### Capsules

A large number of unit capsules are prepared by filling standard two-piece hard gelatin capsules each with 100 milligrams of powdered active ingredient, 150 milligrams of lactose, 50 milligrams of cellulose, and 6 milligrams magnesium stearate.

### Soft Gelatin Capsules

A mixture of active ingredient in a digestable oil such as soybean oil, cottonseed oil or olive oil is prepared and injected by means of a positive displacement pump into gelatin to form soft gelatin capsules containing 100 milligrams of the active ingredient. The capsules are washed and dried.

### Tablets

A large number of tablets are prepared by conventional procedures so that the dosage unit was 100 milligrams of active ingredient, 0.2 milligrams of colloidal silicon dioxide, 5 milligrams of magnesium stearate, 275 milligrams of microcrystalline cellulose, 11 milligrams of starch and 98.8 milligrams of lactose. Appropriate coatings may be applied to increase palatability or delay absorption.

Tables 2 and 3 below set forth representative compounds of the present invention.

In said Tables each of the Examples includes all stereoisomers of the indicated structure.

The structures of the Examples below are shown in Table 1c.

Preparation of the Cyclic Urea (XXXIIIa): 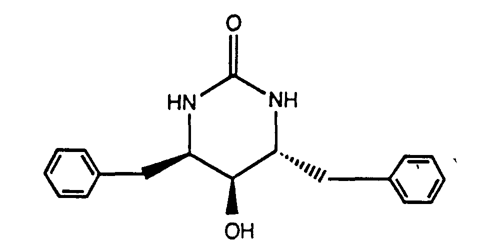
A. Preparation of 4-Amino-2-(t-butoxycarbonylamino)-1,5-diphenyl-3-(2-methoxyethoxymethyl)pentane.
   A mixture of 595 mg (1.50 mmole) of 4-azido-2-(t-butoxycarbonylamino-1,5-diphenyl-3-hydroxypentane (EP 0 402 646 A1), 10 ml of dioxane, 0.2 ml (1.75 mmole) of MEM chloride, and 0.32 ml (1.83 mmole) of diisopropylethylamine was heated at 80°C for 16 h. Evaporated the solvent and purified the residue by flash chromatography on silica gel with 85:15 hexane-ethyl acetate to give 0.64 g (88%) of an oil. Mass spec (M+H)⁺ = 485.2. This was reduced to the title compound with hydrogen using 100 mg of 10 % Pd on carbon in 60 ml of ethyl acetate and 0.6 ml of acetic acid in 49% yield.
B. Preparation of 2,4-diamino-1,5-diphenyl-3-hydroxypentane.
   The product from Part A (218 mg) was dissolved in 2 ml of ice cold 1:1 trifluoroacetic acid - dichloromethane. After 1 h the solution was poured into a mixture of sodium bicarbonate solution and ethyl acetate. The ethyl acetate extract yielded 163 mg of the desired diamino compound.
C. Cyclization of the Diamine
   The product from Part B (146 mg), 75 mg of carbonyl diimidazole, and 0.15 ml of diisopropylethylamine were dissolved in 2.5 ml of anydrous THF and stirred at room temperature for 16 h. The solvent was evaporated. The residue was purified by preparative TLC on silica gel with 90:10 dichloromethane - methanol to give 108 mg (69 %) of the cyclic urea. Mass spec (M+H)⁺ = 385.1.
   N-Alkylation of the Cyclic Urea (XXXIIIa) :
D. The product from Part C (93 mg) was dissolved in 2.5 ml of anhydrous DMF, and 100 mg of 60% NaH in mineral oil was added. The mixture was stirred for one h. m-Benzyloxbenzyl chloride (350 mg) was added, and the mixture was stirred for 16 hrs at room temperature.

Water and ethyl acetate were added. The ethyl acetate extract was washed with water, dried and evaporated . The residue was purified by prep TLC on silica gel with 60:40 hexane - ethyl acetate to give 105 mg (54%) of the desired bis-alkylated product. Mass spec (M+H) + = 777.5

Deprotection of Protecting Groups (Example 22A):

The product from part D (103 mg) was dissolved in 4N HCl/dioxane for 16 h. The solution was evaporated and purified by prep TLC on silica gel with 60:40 hexane - ethyl acetate. Mass spec (M+H)⁺ = 689.4. The purified material was hydrogenated for 16 h in the presence of 3 ml ethanol. 0.2 ml of acetic acid, and 35 mg of 10% Pd on carbon to give Example 22A. Mass spec (M+H)⁺ = 509.25; calculated, 509.24.

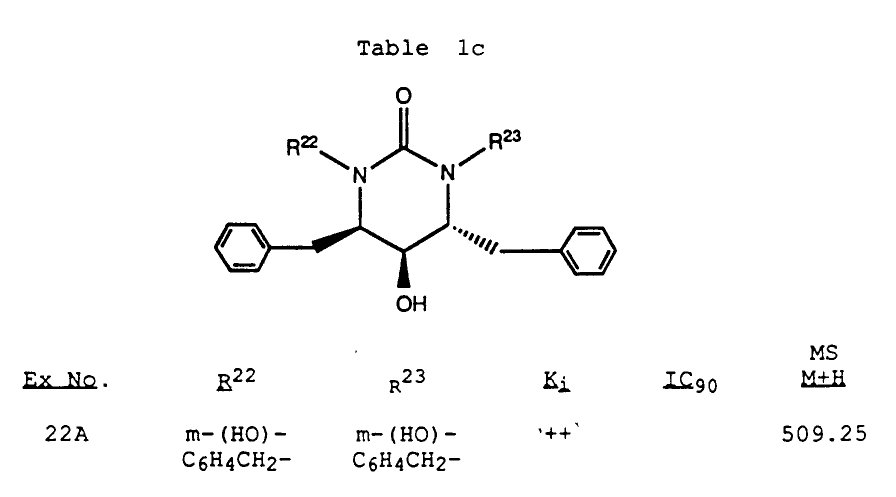

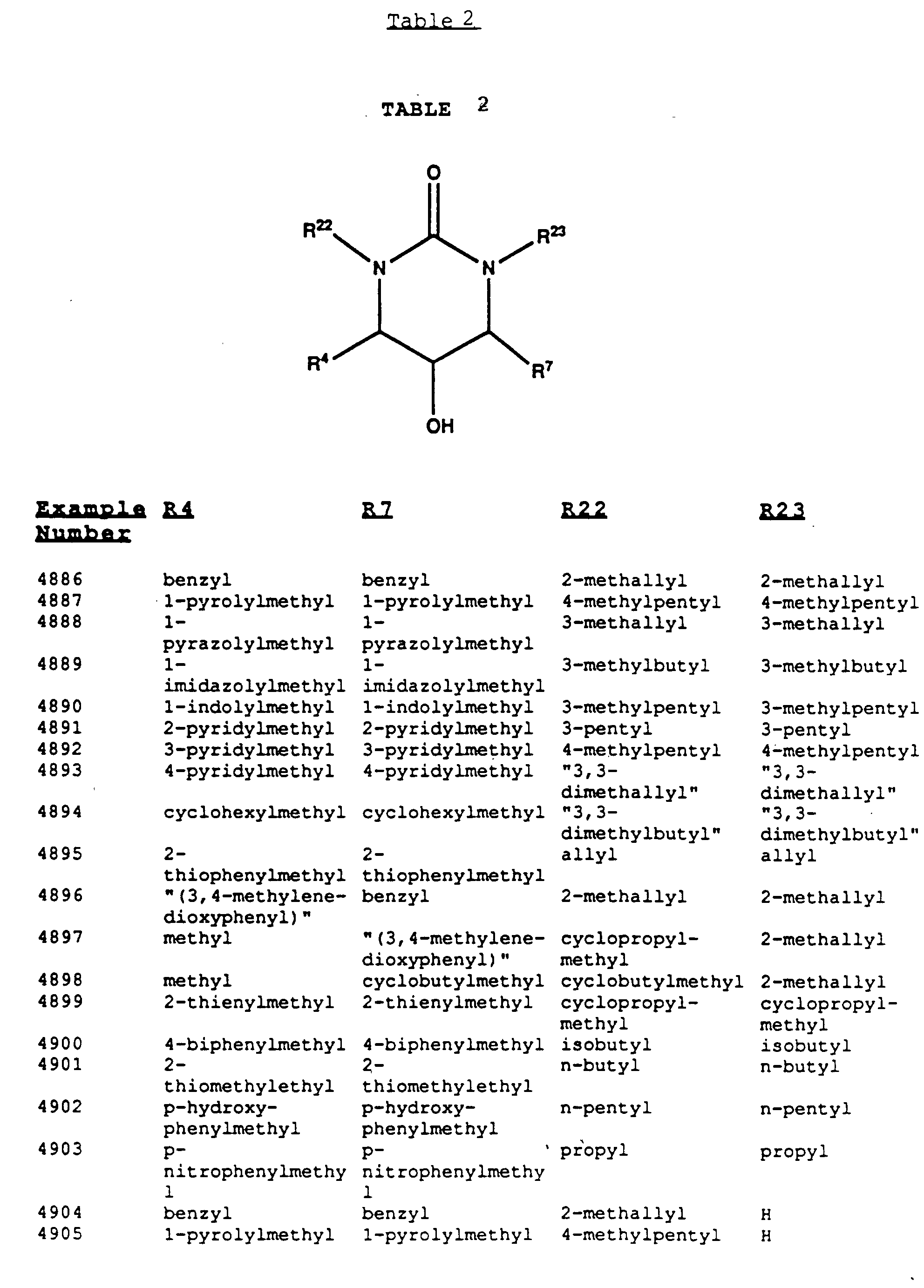

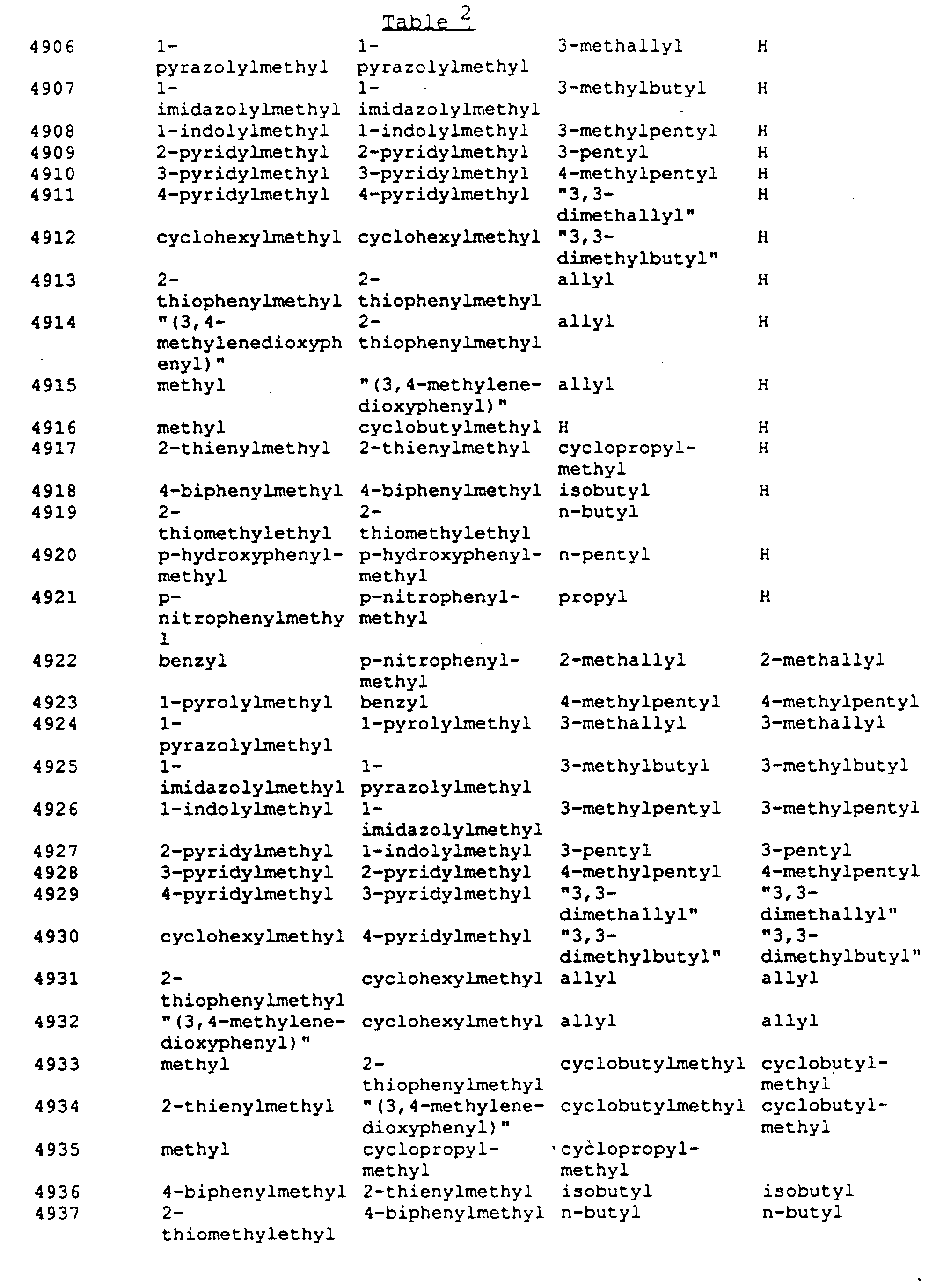

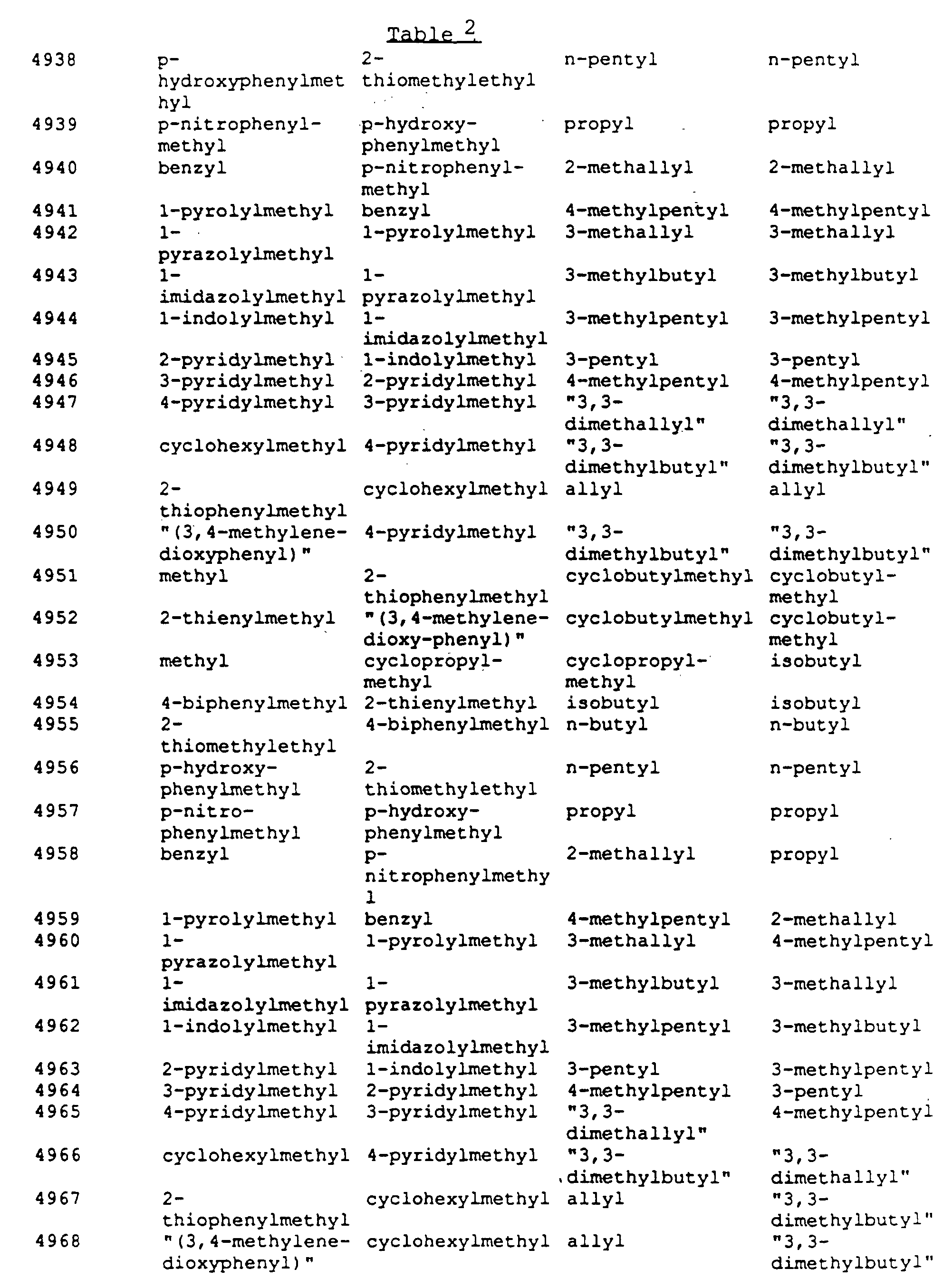

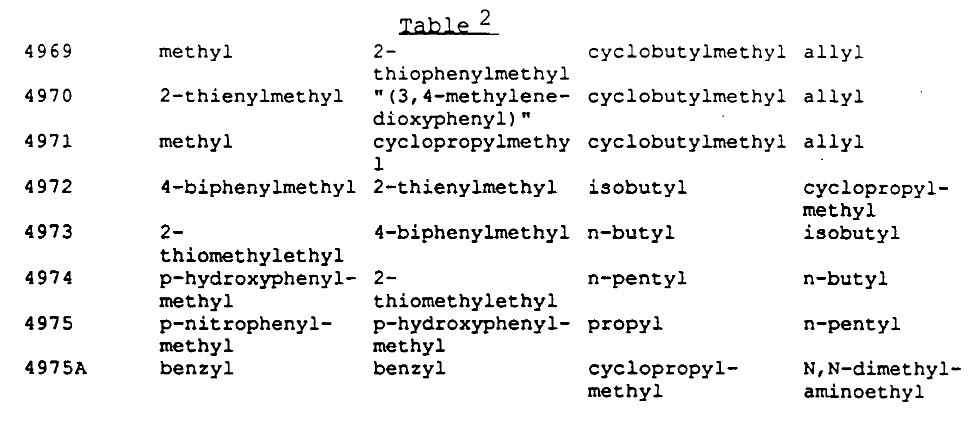

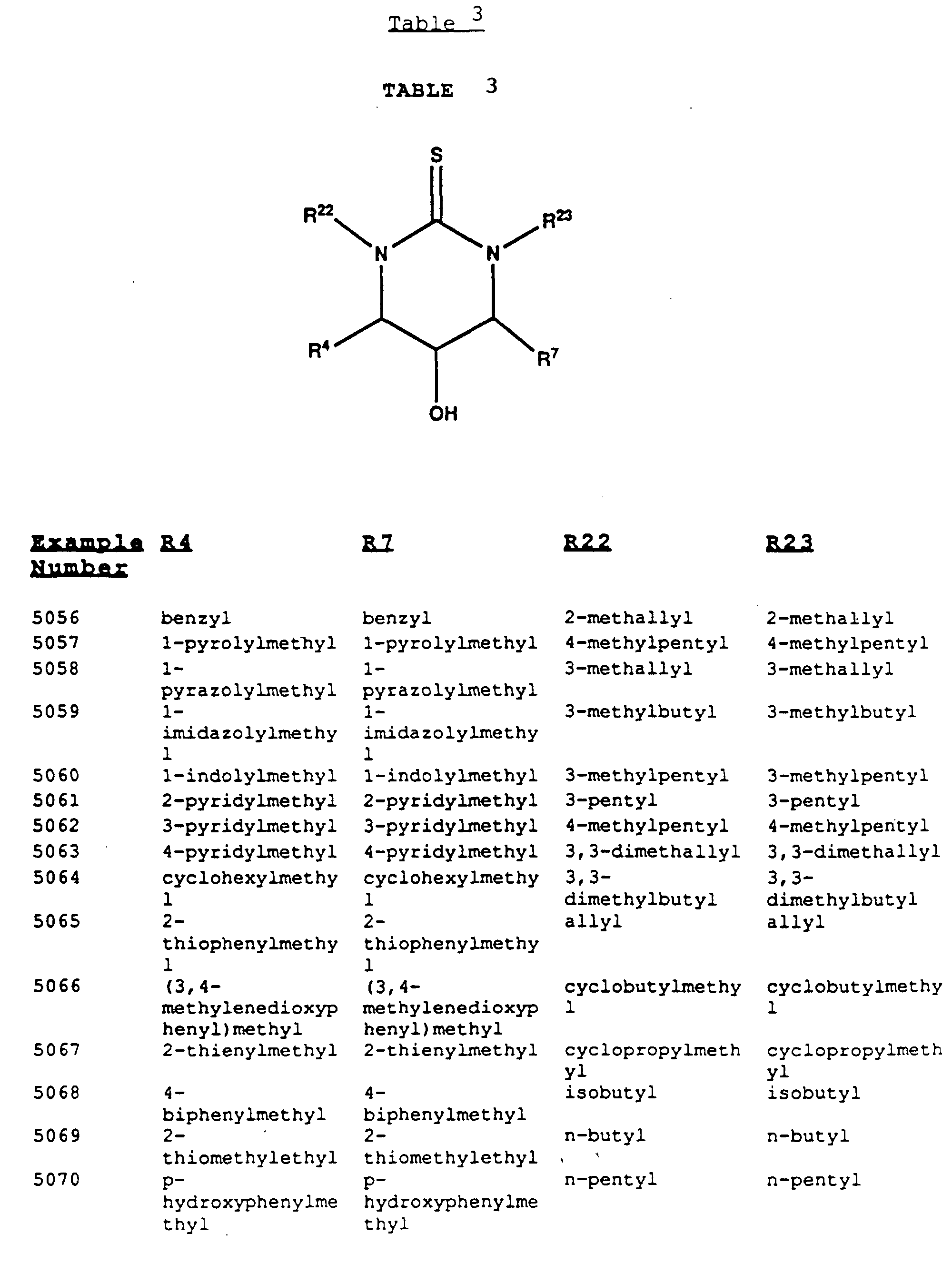

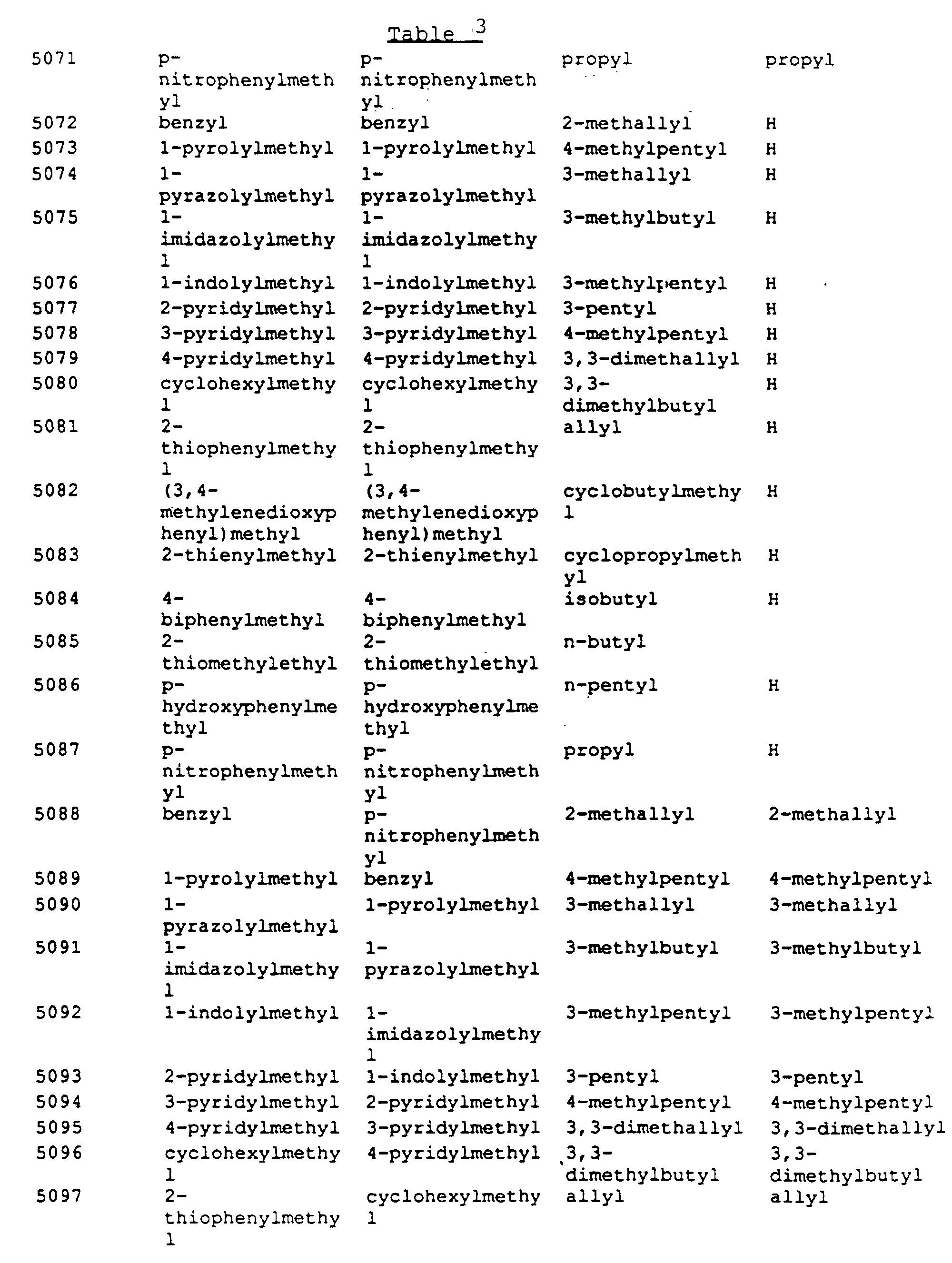

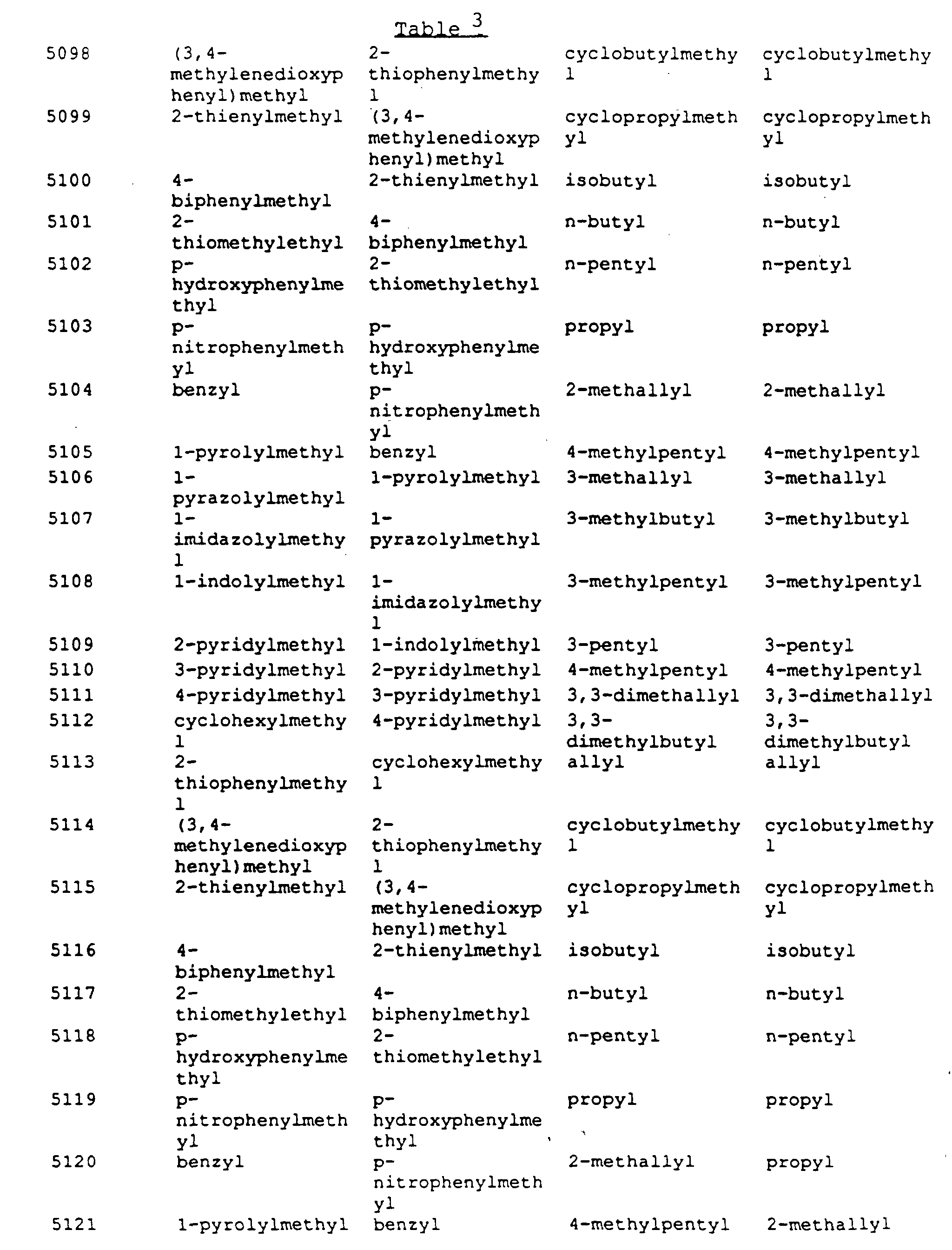

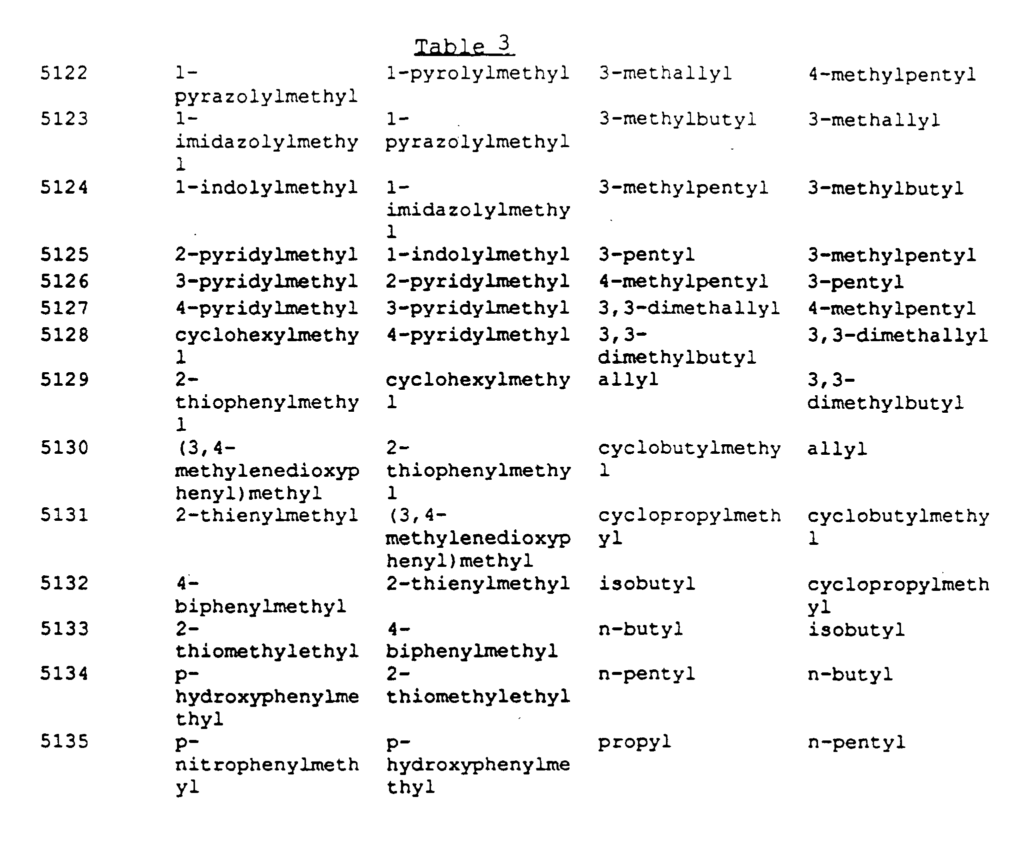

## Claims

1. A compound of the formula (I): 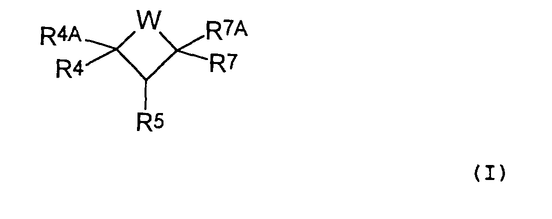 or a pharmaceutically acceptable salt or prodrug form thereof wherein:
R⁴ and R⁷ are independently selected from the following groups:
hydrogen;
C₁-C₈ alkyl substituted with 0-3 R¹¹;
C₂-C₈ alkenyl substituted with 0-3 R¹¹;
C₂-C₈ alkynyl substituted with 0-3 R¹¹;
C₃-C₈ cycloalkyl substituted with 0-3 R¹¹;
C₆-C₁₀ bicycloalkyl substituted with 0-3 R¹¹;
aryl substituted with 0-3 R¹²;
a C₆-C₁₄ carbocyclic residue substituted with 0-3 R¹²;
a heterocyclic ring system substituted with 0-2 R¹², composed of 5 to 10 atoms including at least one nitrogen, oxygen or sulfur atom;
R^{4A} and R^{7A} are independently selected from the following groups:
hydrogen;
C₁-C₄ alkyl substituted with halogen or C₁-C₂ alkoxy;
benzyl substituted with halogen or C₁-C₂ alkoxy;
R⁴ and R^{4A} can alternatively join to form a 5-7 membered carbocyclic ring substituted with 0-2 R¹²;
R⁷ and R^{7A} can alternatively join to form a 5-7 membered carbocyclic ring substituted with 0-2 R¹²;
R⁵ is selected from fluoro, difluoro, =O, and -OR²⁰;
R²⁰ is selected from:
hydrogen;
C₁-C₆ alkyl substituted with 0-3 R¹¹;
C₃-C₆ alkoxyalkyl substituted with 0-3 R¹¹;
C1-C6 alkylcarbonyl substituted with 0-3 R¹¹;
C₁-C₆ alkoxycarbonyl substituted with 0-3 R¹¹;
benzoyl substituted with 0-3 R¹²;
phenoxycarbonyl substituted with 0-3 R¹²;
phenylaminocarbonyl substituted with 0-3 R¹²;
R¹¹ is selected from one or more of the following:
keto, halogen, cyano, -CH₂NR¹³R¹⁴, -NR¹³R¹⁴, -CO₂R¹³, -OC(=O)R¹³, -OR¹³, C₂-C₆ alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
1-3 amino acids, linked together via amide bonds and linked to R⁴ or R⁷ via the amine or carboxylate terminus;
a C₅-C₁₄ carbocyclic residue substituted with 0-3 R¹²;
aryl substituted with 0-3 R¹²;
a heterocyclic ring system substituted with 0-2 R¹², composed of 5 to 10 atoms including at least one nitrogen, oxygen or sulfur atom;
R¹², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy;
a 5- or 6-membered heterocyclic ring containing from 1 to 4 heteroatoms selected from oxygen, nitrogen and sulfur;
or R¹² may be a 3- or 4- carbon chain attached to adjacent carbons on the ring to form a fused 5- or 6- membered ring, said 5- or 6- numbered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl;
R¹³ is H, phenyl, benzyl, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is selected from:
-N(R²²)C(=Z)N(R²³)-;
-N(R²²)S(=O)N(R²³)-;
wherein:
Z is O, S, or NR²⁴;
R²² and R²³ are independently selected from the following:
hydrogen;
C₁-C₈ alkyl substituted with 0-3 R³¹;
C₂-C₈ alkenyl substituted with 0-3 R³¹;
C₂-C₈ alkynyl substituted with 0-3 R³¹;
C₃-C₈ cycloalkyl substituted with 0-3 R³¹;
C₆-C₁₀ bicycloalkyl substituted with 0-3 R³¹;
aryl substituted with 0-3 R³²;
a C₆-C₁₄ carbocyclic residue substituted with 0-3 R³²;
a heterocyclic ring system substituted with 0-2 R³², composed of 5 to 10 atoms including at least one nitrogen, oxygen or sulfur atom;
R²⁴ is selected from: hydroxy; amino; C₁-C₄ alkyl; C₁-C₄ alkoxy; C₁-C₄ aminoalkyl; cyano; nitro; benzyloxy;
alternatively, R²² can join with R⁴ or R^{4A} to form a five- or six-membered fused heterocyclic or carbocyclic ring substituted with 0-2 R¹²; and
alternatively, R²³ can join with R⁷ or R^{7A} to form a five- or six-membered fused heterocyclic or carbocyclic ring substituted with 0-2 R¹²; and
alternatively, W can join with R⁵ to form a three-to seven-membered fused heterocyclic or carbocyclic ring substituted with 0-2 R¹²;
R³¹ is selected from one or more of the following:
keto, halogen, cyano, -CH₂NR¹³R¹⁴, -NR¹³R¹⁴, -CO₂R¹³, -OC(=O)R¹³, -OR¹³, C₂-C₆ alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄ alkyl, C₂-C₄ alkenyl, C₃-C₆ cycloalkyl, C₃-C₆ cycloalkylmethyl;
1-3 amino acids, linked together via amide bonds and linked to R⁴ or R⁷ via the amine or carboxylate terminus;
a C₅-C₁₄ carbocyclic residue substituted with 0-3 R³²;
aryl substituted with 0-3 R³²; or
a heterocyclic ring system substituted with 0-2 R³², composed of 5 to 10 atoms including at least one nitrogen, oxygen or sulfur atom;
R³², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -OCH₂CO₂H, 2-(1-morpholino)ethoxy, -C(R¹⁴)=N(OR¹⁴); or
a 5- or 6-membered heterocyclic ring containing from 1 to 4 heteroatoms selected from oxygen, nitrogen and sulfur;
or R³² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R³² is attached to a saturated carbon atom, it may be carbonyl or thiocarbonyl;
R³², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, -CO₂H, C₁-C₄ alkylcarbonyloxy, C₁-C₄ alkylcarbonyl, -C(R¹⁴)=N(OR¹⁴) ;
provided that:
R⁴, R^{4A}, R⁷, and, R^{7A} are not all hydrogen;
when R⁴ and R^{4A} are both hydrogen, at least one of R²² and R²³ is not hydrogen.

2. The compound of claim 1 wherein:
R⁴ and R⁷ are independently selected from the following groups:
hydrogen;
C₁-C₄ alkyl substituted with 0-3 R¹¹;
C₃-C₄ alkenyl substituted with 0-3 R¹¹;
C₃-C₄ alkynyl substituted with 0-3 R¹¹;
R^{4A} and R^{7A} are hydrogen;
R²⁰ is selected from:
hydrogen;
C1-C6 alkylcarbonyl;
C₁-C₆ alkoxycarbonyl;
benzoyl;
R¹¹ is selected from one or more of the following:
keto, halogen, cyano, -CH₂NR¹³R¹⁴, -NR¹³R¹⁴, -CO₂R¹³,
-OC(=O)R¹³, -OR¹³, C₂-C₄ alkoxyalkyl, -S(O)ₘR¹³, C₁-C₄ alkyl, C₂-C₄ alkenyl, C₃-C₆ cycloalkyl; a C₅-C₁₄ carbocyclic residue substituted with 0-3 R¹²;
aryl substituted with 0-3 R¹²;
a heterocyclic ring system substituted with 0-2 R¹², composed of 5 to 10 atoms including at least one nitrogen, oxygen or sulfur atom;
R¹², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴; or
a 5- or 6-membered heterocyclic ring containing from 1 to 4 heteroatoms selected from oxygen, nitrogen or sulfur;
or R¹² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R¹² is attached to a saturated carbon atom, it may be carbonyl or thiocarbonyl;
R¹², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H;
R¹³ is H, C₁-C₆ alkyl, or C₃-C₆ alkoxyalkyl;
R¹⁴ is OH, H, C₁-C₄ alkyl, or benzyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R¹⁵ is H or CH₃;
m is 0, 1 or 2;
W is -
-N(R²²)C(=Z)N(R²³)-;
wherein:
Z is O, S, N-CN, N-OH, N-OCH₃;
R²² and R²³ are independently selected from the following:
hydrogen;
C₁-C₈ alkyl substituted with 0-3 R³¹;
C₃-C₈ alkenyl substituted with 0-3 R³¹;
C₃-C₈ alkynyl substituted with 0-3 R³¹;
C₃-C₆ cycloalkyl substituted with 0-3 R³¹;
R³¹ is selected from one or more of the following:
keto, halogen, cyano, -CH₂NR¹³R¹⁴, -NR¹³R¹⁴, -CO₂R¹³, -OC(=O)R¹³, -OR¹³, C₂-C₄ alkoxyalkyl, -S(O)ₘR¹³, C₁-C₄ alkyl, C₂-C₄ alkenyl, C₃-C₆ cycloalkyl;
a C₅-C₁₄ carbocyclic residue substituted with 0-3 R¹²;
aryl substituted with 0-3 R³²;
a heterocyclic ring system substituted with 0-2 R³², composed of 5 to 10 atoms including at least one nitrogen, oxygen or sulfur atom;
R³², when a substituent on carbon, is selected from one or more of the following:
phenyl, benzyl, phenethyl, phenoxy, benzyloxy, halogen, hydroxy, nitro, cyano, C₁-C₄ alkyl, C₃-C₆ cycloalkyl, C₃-C₆ cycloalkylmethyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -S(O)ₘR¹³, -SO₂NR¹³R¹⁴, -NHSO₂R¹⁴, -C(R¹⁴)=N(OR¹⁴);
a 5- or 6-membered heterocyclic ring containing from 1 to 4 heteroatoms selected from oxygen, nitrogen and sulfur;
or R³² may be a 3- or 4- carbon chain attached to adjacent carbons on the ring to form a fused 5- or 6-membered ring, said 5- or 6- membered ring being optionally substituted on the aliphatic carbons with halogen, C₁-C₄ alkyl, C₁-C₄ alkoxy, hydroxy, or -NR¹³R¹⁴; or, when R³² is attached to a saturated carbon atom, it may be carbonyl or thiocarbonyl;
R³², when a substituent on nitrogen, is selected from one or more of the following:
phenyl, benzyl, phenethyl, hydroxy, C₁-C₄ hydroxyalkyl, C₁-C₄ alkoxy, C₁-C₄ alkyl, C₃-C₆ cycloalkyl, C₃-C₆ cycloalkylmethyl, -CH₂NR¹³R¹⁴, -NR¹³R¹⁴, C₂-C₆ alkoxyalkyl, C₁-C₄ haloalkyl, C₁-C₄ alkoxycarbonyl, C₁-C₄ alkylcarbonyloxy, C₁-C₄ alkylcarbonyl, -CO₂H, -C(R¹⁴)=N(OR¹⁴);
provided that:
R⁴, R^{4A}, R⁷, and R^{7A} are not all hydrogen;
when R⁴ and R^{4A} are both hydrogen, at least one of R²² and R²³ is not hydrogen.

3. The compound of claim 1 having the formula (II): 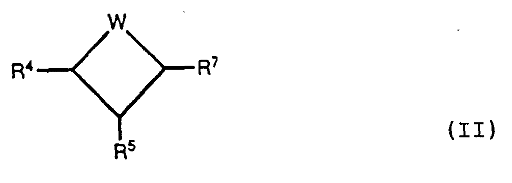 or a pharmaceutically acceptable salt or prodrug form thereof wherein:
R⁴ and R⁷ are independently selected from the following groups:
hydrogen;
C₁-C₄ alkyl substituted with 0-3 R¹¹;
C₃-C₄ alkenyl substituted with 0-3 R¹¹;
R⁵ is -OR²⁰;
R²⁰ is as defined in claim 1;
R¹¹ is selected from one or more of the following:
keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
aryl substituted with 0-3 R¹²;
a heterocyclic ring system substituted with 0-2 R¹², composed of 5 to 10 atoms including at least one nitrogen, oxygen or sulfur atom;
R¹², when a substituent on carbon, is selected from one or more of the following:
phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, methylenedioxy, C₁-C₄ haloalkyl, C₁-C₄ alkylcarbonyl, C₁-C₄ alkylcarbonylamino, hydroxy, hydroxymethyl; or
a 5- or 6-membered heterocyclic ring containing from 1 to 4 heteroatoms selected from oxygen, nitrogen or sulfur;
R¹², when a substituent on nitrogen, is selected from benzyl or methyl;
R¹³ is H, C₁-C₂ alkyl, or C₃-C₆ alkoxyalkyl;
R¹⁴ is OH, H or C₁-C₂ alkyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
W is -N(R²²)C(=Z)N(R²³)-;
wherein:
Z is O, S, or N-CN;
R²² and R²³ are independently selected from the following:
hydrogen;
C₁-C₄ alkyl substituted with 0-3 R³¹;
C₃-C₄ alkenyl substituted with 0-3 R³¹;
R³¹ is selected from one or more of the following:
keto, halogen, -CH₂NR¹³R¹⁴, -NR¹³R¹⁴, -OR¹³, C₂-C₄ alkoxyalkyl, C₁-C₄ alkyl, C₂-C₄ alkenyl, C₃-C₆ cycloalkyl;
aryl substituted with 0-3 R³²; or
a heterocyclic ring system substituted with 0-2 R³², composed of 5 to 10 atoms including at least one nitrogen, oxygen or sulfur atom;
R³², when a substituent on carbon, is selected from one or more of the following:
phenyl, benzyl, phenethyl, phenoxy, benzyloxy, halogen, C₁-C₄ alkyl, C₇-C₁₀ arylalkyl, C₁-C₄ alkoxy, -CO₂H, hydroxamic acid, hydrazide, oxime, boronic acid, sulfonamide, formyl, C₃-C₆ cycloalkoxy, -OR¹³, C₁-C₄ alkyl substituted with -NR¹³R¹⁴, -NR¹³R¹⁴, methylenedioxy, C₁-C₄ haloalkyl, C₁-C₄ alkylcarbonyl, C₁-C₄ alkylcarbonylamino, hydroxy, hydroxymethyl, -C(R¹⁴)=N(OR¹⁴);
a 5- or 6-membered heterocyclic ring containing from 1 to 4 heteroatoms selected from oxygen, nitrogen and sulfur;
R³², when a substituent on nitrogen, is benzyl or methyl;
provided that:
when R⁴ is hydrogen, R⁷ is not hydrogen;
when R⁴ is hydrogen, at least one of R²² and R²³ is not hydrogen.

4. The compound of claim 1 having the formula (III): 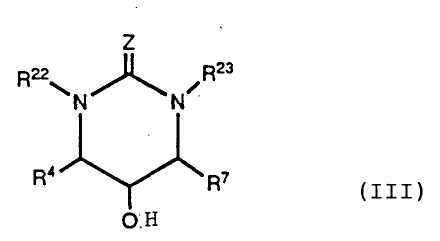 wherein:
R⁴ and R⁷ are independently selected from the following groups:
hydrogen;
C₁-C₃ alkyl substituted with 0-3 R¹¹;
R¹¹ is selected from one or more of the following:
halogen, -OR¹³, C₁-C₄ alkyl, C₃-C₅ cycloalkyl;
aryl substituted with 0-2 R¹²;
a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R¹², when a substituent on carbon, is selected from one or more of the following:
benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl; or
R¹², when a substituent on nitrogen, is methyl;
R¹³ is H or methyl;
R¹⁴ is OH, H or methyl;
R¹³ and R¹⁴ can alternatively join to form -(CH₂)₄-, -(CH₂)₅-, -CH₂CH₂N(R¹⁵)CH₂CH₂-, or -CH₂CH₂OCH₂CH₂-;
R²² and R²³ are independently selected from the following:
hydrogen;
C₁-C₄ alkyl substituted with 0-1 R³¹;
C₃-C₄ alkenyl substituted with 0-1 R³¹;
R³¹ is selected from one or more of the following:
halogen, -OR¹³, C₁-C₄ alkyl, C₃-C₅ cycloalkyl;
aryl substituted with 0-2 R³²;
a heterocyclic ring system chosen from pyridyl, pyrimidinyl, triazinyl, furanyl, thienyl, pyrrolyl, pyrazolyl, imidazolyl, tetrazolyl, benzofuranyl, indolyl, quinolinyl, isoquinolinyl;
R³², when a substituent on carbon, is selected from one or more of the following:
benzyloxy, halogen, methyl, C₁-C₄ alkoxy, CF₃, 2-(1-morpholino)ethoxy, -CO₂H, hydroxamic acid, hydrazide, oxime, cyano, boronic acid, sulfonamide, formyl, C₃-C₆ cycloalkoxy, C₁-C₄ alkyl substituted with -NR¹³R¹⁴, -NR¹³R¹⁴, hydroxy, hydroxymethyl, -C(R¹⁴)=N(OR¹⁴) ; or
R³², when a substituent on nitrogen, is methyl;
provided that:
when R⁴ is hydrogen, R⁷ is not hydrogen;
when R⁴ is hydrogen, at least one of R²² and R²³ is not hydrogen.

5. The compound of claim 1 having the formula (IV): 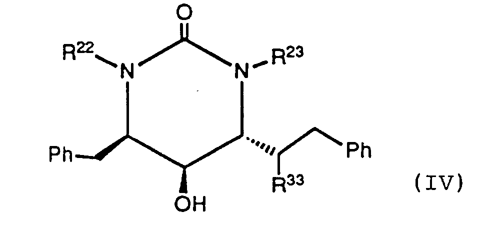 or a pharmaceutically acceptable salt or prodrug form thereof wherein:
R³³ is OH, halogen, H, N₃, or can alternatively be taken together with R²³ to form a direct bond;
R²² and R²³ are independently selected from:
hydrogen, allyl, propyl, cyclopropylmethyl, n-butyl, i-butyl, CH₂CH=CH(CH₃)₂, pyridylmethyl, methallyl, n-pentyl, i-pentyl, hexyl, benzyl, pyridylmethyl, isoprenyl, propargyl, picolinyl, methoxyethyl, cyclohexylmethyl, dimethyl-butyl, ethoxyethyl, methyloxazolinylmethyl, naphthylmethyl, methyloxazolinylmethyl, vinyloxyethyl, pentafluorobenzyl, quinolinylmethyl, carboxybenzyl, chloro-thienyl, picolinyl, benzyloxybenzyl, phenylbenzyl, adamantylethyl, cyclopropylmethoxybenzyl, ethoxybenzyl, hydroxybenzyl, hydroxymethylbenzyl, aminobenzyl, formylbenzyl, cyanobenzyl, cinnamyl, allyloxybenzyl, fluorobenzyl, cyclobutylmethyl, formaldoximebenzyl, cyclopentylmethyl, nitrobenzyl, nitrilobenzyl, carboxamidobenzyl, carbomethoxybenzyl, and dimethylallyl.

6. Use of a compound according to any one of claims 1 to 5 for the preparation of a medicament for the treatment of viral infections.

7. A pharmaceutical composition comprising a pharmaceutical acceptable carrier and at least one compound. according to any one of claims 1 to 5.

## Patentansprüche

1. Verbindung der Formel (I): 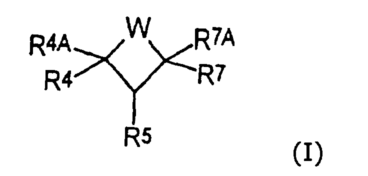 oder ein pharmazeutisch annehmbares Salz oder eine Medikamentenvorstufe (Prodrug) derselben, wobei
R⁴ und R⁷ unabhängig voneinander aus den folgenden Gruppen ausgewählt sind:
Wasserstoff,
C₁-C₈-Alkyl, das mit 0-3 R¹¹ substituiert ist;
C₂-C₈-Alkenyl, das mit 0-3 R¹¹ substituiert ist;
C₂-C₈-Alkinyl, das mit 0-3 R¹¹ substituiert ist;
C₃-C₈-Cycloalkyl, das mit 0-3 R¹¹ substituiert ist;
C₆-C₁₀-Bicycloalkyl, das mit 0-3 R¹¹ substituiert ist;
Aryl, das mit 0-3 R¹² substituiert ist;
einem carbocyclischen C₆-C₁₄-Rest, der mit 0-3 R¹² substituiert ist;
einem heterocyclischen Ringsystem, das mit 0-2 R¹² substituiert ist, bestehend aus 5 bis 10 Atomen, die wenigstens ein Stickstoff-, Sauerstoff- oder Schwefelatom einschließen;
R^{4A} und R^{7A} unabhängig voneinander aus den folgenden Gruppen ausgewählt sind:
Wasserstoff
C₁-C₄-Alkyl, das mit Halogen oder C₁-C₂-Alkoxy substituiert ist;
Benzyl, das mit Halogen oder C₁-C₂-Alkoxy substituiert ist;
R⁴ und R^{4A} sich alternativ unter Bildung eines fünf- bis siebengliedrigen carbocyclischen Rings verbinden können, der mit 0-2 R¹² substituiert ist;
R⁷ und R^{7A} sich alternativ unter Bildung eines fünf- bis siebengliedrigen carbocyclischen Rings verbinden können, der mit 0-2 R¹² substituiert ist;
R⁵ aus Fluor, Difluor, =O und -OR²⁰ ausgewählt ist;
R₂₀ aus
Wasserstoff;
C₁-C₆-Alkyl, das mit 0-3 R¹¹ substituiert ist;
C₃-C₆-Alkoxyalkyl, das mit 0-3 R¹¹ substituiert ist;
C₁-C₆-Alkylcarbonyl, das mit 0-3 R¹¹ substituiert ist;
C₁-C₆-Alkoxycarbonyl, das mit 0-3 R¹¹ substituiert ist;
Benzoyl, das mit 0-3 R¹² substituiert ist;
Phenoxycarbonyl, das mit 0-3 R¹² substituiert ist;
Phenylaminocarbonyl, das mit 0-3 R¹² substituiert ist;
ausgewählt ist;
R¹¹ aus einer oder mehreren der folgenden Gruppen ausgewählt ist:
Keto, Halogen, Cyano, -CH₂NR¹³R¹⁴, -NR¹³R¹⁴, -CO₂R¹³, -OC(=O)R¹³, -OR¹³, C₂-C₆-Alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄-Alkyl, C₂-C₄-Alkenyl, C₃-C₆-Cycloalkyl, C₃-C₆-Cycloalkylmethyl;
1 bis 3 Aminosäuren, die über Amidbindungen miteinander verbunden sind und über den Amin- oder Carboxylat-Terminus an R⁴ oder R⁷ gebunden sind;
einem carbocyclischen C₅-C₁₄-Rest, der mi 0-3 R¹² substituiert ist;
Aryl, das mit 0-3 R¹² substituiert ist;
einem heterocyclischen Ringsystem, das mit 0-2 R¹² substituiert ist, bestehend aus 5 bis 10 Atomen, die wenigstens ein Stickstoff-, Sauerstoff- oder Schwefelatom einschließen;
R¹² aus einer oder mehreren der folgenden Gruppen ausgewählt ist, wenn es ein Substituent am Kohlenstoff ist:
Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Boronsäure, Sulfonamid, Formyl, C₃-C₆-Cycloalkoxy, -OR¹³, C₁-C₄-Alkyl, das mit -NR¹³R¹⁴ substituiert ist, -NR¹³R¹⁴, C₂-C₆-Alkoxyalkyl, C₁-C₄-Hydroxyalkyl, Methylendioxy, Ethylendioxy, C₁-C₄-Haloalkyl, C₁-C₄-Haloalkoxy, C₁-C₄-Alkoxycarbonyl, C₁-C₄-Alkylcarbonyloxy, C₁-C₄-Alkylcarbonyl, C₁-C₄-Alkylcarbonylamino, -S(O)ₘR¹³, -SO₂NR¹³R¹⁴, -NHSO₂R¹⁴, -OCH₂CO₂H, 2-(1-Morpholino)ethoxy;
einem fünf- oder sechsgliedrigen heterocyclischen Ring, der 1 bis 4 Heteroaotme enthält, die aus Sauerstoff, Stickstoff und Schwefel ausgewählt sind;
oder R¹² eine drei - oder viergliedrige Kohlenstoffkette sein kann, die an benachbarte Kohlenstoffe am Ring gebunden ist, um einen anellierten fünf- oder sechsgliedrigen Ring zu bilden, wobei der fünf- oder sechsgliedrige Ring gegebenenfalls mit Halogen, C₁-C₄-Alkyl, C₁-C₄-Alkoxy, Hydroxy oder -NR¹³R¹⁴ an den aliphatischen Kohlenstoffen substituiert ist;
oder wenn R¹² an ein gesättigtes Kohlenstoffatom gebunden ist, es Carbonyl oder Thiocarbonyl sein kann;
R¹² aus einer oder mehreren der folgenden Gruppen ausgewählt ist, wenn es ein Substituent am Stickstoff ist:
Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Haloalkyl, C₁-C₄-Alkoxycarbonyl, -CO₂H, C₁-C₄-Alkylcarbonyloxy, C₁-C₄-Alkylcarbonyl;
R¹³ H, Phenyl, Benzyl, C₁-C₆-Alkyl oder C₃-C₆-Alkoxyalkyl ist;
R¹⁴ OH, H, C₁-C₄-Alkyl oder Benzyl ist;
R¹³ und R¹⁴ sich alternativ verbinden können, um -(CH₂)₄-, -(CH₂)₅-, -CH₂CH₂N(R¹⁵)CH₂CH₂- oder -CH₂CH₂OCH₂CH₂- zu bilden;
R¹⁵ H oder CH₃ ist;
m 0, 1 oder 2 ist;
W aus:
-N(R²²)C(=Z)N(R²³)-,
-N(R²²)S(=O)N(R²³)-
ausgewählt ist; wobei Z O, S oder NR²⁴ ist;
R²² und R²³ unabhängig voneinander aus den folgenden Gruppen ausgewählt sind:
Wasserstoff,
C₁-C₈-Alkyl, das mit 0-3 R³¹ substituiert ist;
C₂-C₈-Alkenyl, das mit 0-3 R³¹ substituiert ist;
C₂-C₈-Alkinyl, das mit 0-3 R³¹ substituiert ist;
C₃-C₈-Cycloalkyl, das mit 0-3 R³¹ substituiert ist;
C₆-C₁₀-Bicycloalkyl, das mit 0-3 R³¹ substituiert ist;
Aryl, das mit 0-3 R³² substituiert ist;
einem carbocyclischen C₆-C₁₄-Rest, der mit 0-3 R³² substituiert ist;
einem heterocyclischen Ringsystem, das mit 0-2 R³² substituiert ist, bestehend aus 5 bis 10 Atomen, die wenigstens ein Stickstoff-, Sauerstoff- oder Schwefelatom einschließen;
R²⁴ aus Hydroxy, Amino, C₁-C₄-Alkyl, C₁-C₄-Alkoxy, C₁-C₄-Aminoalkyl, Cyano, Nitro, Benzyloxy ausgewählt ist; alternativ dazu R²² sich mit R⁴ oder R^{4A} verbinden kann, um einen fünf- oder sechsgliedrigen, anellierten, heterocyclischen Ring oder carbocyclischen Ring zu bilden, der mit 0-2 R¹² substituiert ist; und
R²³ sich alternativ dazu mit R⁷ oder R^{7A} verbinden kann, um einen fünf- oder sechsgliedrigen, anellierten, heterocyclischen Ring oder carbocyclischen Ring zu bilden, der mit 0-2 R¹² substituiert ist; und
W sich alternativ dazu mit R⁵ verbinden kann, um einen drei- bis siebengliedrigen, anellierten, heterocyclischen Ring oder carbocyclischen Ring zu bilden, der mit 0-2 R¹² substituiert ist;
R³¹ aus einer oder mehreren der folgenden Gruppen ausgewählt ist:
Keto, Halogen, Cyano, -CH₂NR¹³R¹⁴, -NR¹³R¹⁴, -CO₂R¹³, -OC(=O)R¹³, -OR¹³, C₂-C₆-Alkoxyalkyl, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, C₁-C₄-Alklyl, C₂-C₄-Alkenyl, C₃-C₆-Cycloalkyl, C₃-C₆-Cycloalkylmethyl;
1 bis 3 Aminosäuren, die über Amidbindungen miteinander verbunden sind und über den Amin- oder Carboxylat-Terminus an R⁴ oder R⁷ gebunden sind;
einem carbocyclischen C₅-C₁₄-Rest, der mit 0-3 R³² substituiert ist;
Aryl, das mit 0-3 R³² substituiert ist; oder
einem heterocyclischen Ringsystem, das mit 0-2 R³² substituiert ist, bestehend aus 5 bis 10 Atomen, die wenigstens ein Stickstoff-, Sauerstoff- oder Schwefelatom einschließen;
R³² aus einer oder mehreren der folgenden Gruppen ausgewählt ist, wenn es ein Substituent am Kohlenstoff ist:
Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Boronsäure, Sulfonamid, Formyl, C₃-C₆-Cycloalkoxy, -OR¹³, C₁-C₄-Alkyl, das mit -NR¹³R¹⁴ substituiert ist, -NR¹³R¹⁴, C₂-C₆-Alkoxyalkyl, C₁-C₄-Hydroxyalkyl, Methylendioxy, Ethylendioxy, C₁-C₄-Haloalkyl, C₁-C₄-Haloalkoxy, C₁-C₄-Alkoxycarbonyl, C₁-C₄-Alkylcarbonyloxy, C₁-C₄-Alkylcarbonyl, C₁-C₄-Alkylcarbonylamino, -S(O)ₘR¹³, -SO₂NR¹³R¹⁴, -NHSO₂R¹⁴, -OCH₂CO₂H, 2-(1-Morpholino)ethoxy, -C(R¹⁴)=N(OR¹⁴); oder
einem fünf- oder sechsgliedrigen heterocyclischen Ring, der 1 bis 4 Heteroaotme enthält, die aus Sauerstoff, Stickstoff und Schwefel ausgewählt sind;
oder R³² eine drei - oder viergliedrige Kohlenstoffkette sein kann, die an benachbarte Kohlenstoffe am Ring gebunden ist, um einen anellierten fünf- oder sechsgliedrigen Ring zu bilden, wobei der fünf- oder sechsgliedrigen Ring gegebenenfalls mit Halogen, C₁-C₄-Alkyl, C₁-C₄-Alkoxy, Hydroxy oder -NR¹³R¹⁴ an den aliphatischen Kohlenstoffen substituiert ist;
oder wenn R³² an ein gesättigtes Kohlenstoffatom gebunden ist, es Carbonyl oder Thiocarbonyl sein kann;
R³² aus einer oder mehreren der folgenden Gruppen ausgewählt ist, wenn es ein Substituent am Stickstoff ist:
Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Haloalkyl, C₁-C₄-Alkoxycarbonyl, -CO₂H, C₁-C₄-Alkylcarbonyloxy, C₁-C₄-Alkylcarbonyl, -C(R¹⁴) =N(OR¹⁴);
mit der Maßgabe, dass:
R⁴, R^{4A}, R⁷ und R^{7A} nicht alle Wasserstoff sind;
wenn R⁴ und R^{4A} beide Wasserstoff sind, wenigstens eines von R²² und R²³ kein Wasserstoff ist.

2. Verbindung gemäß Anspruch 1, worin
R⁴ und R⁷ unabhängig voneinander aus den folgenden Gruppen ausgewählt sind:
Wasserstoff,
C₁-C₄-Alkyl, das mit 0-3 R¹¹ substituiert ist;
C₃-C₄-Alkenyl, das mit 0-3 R¹¹ substituiert ist;
C₃-C₄-Alkinyl, das mit 0-3 R¹¹ substituiert ist;
R^{4A} und R^{7A} Wasserstoff sind;
R²⁰ aus
Wasserstoff,
C₁-C₆-Alkylcarbonyl;
C₁-C₆-Alkoxycarbonyl
Benzoyl
ausgewählt ist;
R¹¹ aus einer oder mehreren der folgenden Gruppen ausgewählt ist:
Keto, Halogen, Cyano, -CH₂NR¹³R¹⁴, -NR¹³R¹⁴, -CO₂R¹³, -OC(=O)R¹³, -OR¹³, C₂-C₄-Alkoxyalkyl, -S(O)ₘR¹³, C₁-C₄-Alkyl, C₂-C₄-Alkenyl, C₃-C₆-Cycloalkyl;
einem carbocyclischen C₅-C₁₄-Rest, der mit 0-3 R¹² substituiert ist;
Aryl, das mit 0-3 R¹² substituiert ist;
einem heterocyclischen Ringsystem, das mit 0-2 R¹² substituiert ist, bestehend aus 5 bis 10 Atomen, die wenigstens ein Stickstoff-, Sauerstoff- oder Schwefelatom einschließen;
R¹² aus einer oder mehreren der folgenden Gruppen ausgewählt ist, wenn es ein Substituent am Kohlenstoff ist:
Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Boronsäure, Sulfonamid, Formyl, C₃-C₆-Cycloalkoxy, -OR¹³, C₁-C₄-Alkyl, das mit -NR¹³R¹⁴ substituiert ist, -NR¹³R¹⁴, C₂-C₆-Alkoxyalkyl, C₁-C₄-Hydroxyalkyl, Methylendioxy, Ethylendioxy, C₁-C₄-Haloalkyl, C₁-C₄-Haloalkoxy, C₁-C₄-Alkoxycarbonyl, C₁-C₄-Alkylcarbonyloxy, C₁-C₄-Alkylcarbonyl, C₁-C₄-Alkylcarbonylamino, -S(O)ₘR¹³, -SO₂NR¹³R¹⁴, -NHSO₂R¹⁴; oder
einem fünf- oder sechsgliedrigen heterocyclischen Ring, der 1 bis 4 Heteroaotme enthält, die aus Sauerstoff, Stickstoff und Schwefel ausgewählt sind;
oder R¹² eine drei - oder viergliedrige Kohlenstoffkette sein kann, die an benachbarte Kohlenstoffe am Ring gebunden ist, um einen anellierten fünf- oder sechsgliedrigen Ring zu bilden, wobei der fünf- oder sechsgliedrige Ring gegebenenfalls mit Halogen, C₁-C₄-Alkyl, C₁-C₄-Alkoxy, Hydroxy oder -NR¹³R¹⁴ an den aliphatischen Kohlenstoffen substituiert ist;
oder wenn R¹² an ein gesättigtes Kohlenstoffatom gebunden ist, es Carbonyl oder Thiocarbonyl sein kann;
R¹² aus einer oder mehreren der folgenden Gruppen ausgewählt ist, wenn es ein Substituent am Stickstoff ist:
Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Haloalkyl, C₁-C₄-Alkoxycarbonyl, C₁-C₄-Alkylcarbonyloxy, C₁-C₄-Alkylcarbonyl, -CO₂H;
R¹³ H, C₁-C₆-Alkyl oder C₃-C₆-Aloxyalkyl ist;
R¹⁴ OH, H, C₁-C₄-Alkyl oder Benzyl ist;
R¹³ und R¹⁴ sich alternativ verbinden können, um -(CH₂)₄-, -(CH₂)₅-, -CH₂CH₂N(R¹⁵)CH₂CH₂- oder -CH₂CH₂OCH₂CH₂- zu bilden;
R¹⁵ H oder CH₃ ist;
m 0, 1 oder 2 ist;
W -N(R²²)C(=Z)N(R²³)- ist,
wobei Z O, S, N-CN, N-OH, N-OCH₃ ist;
R²² und R²³ unabhängig voneinander aus den folgenden Gruppen ausgewählt sind:
Wasserstoff,
C₁-C₈-Alkyl, das mit 0-3 R³¹ substituiert ist;
C₃-C₈-Alkenyl, das mit 0-3 R³¹ substituiert ist;
C₃-C₈-Alkinyl, das mit 0-3 R³¹ substituiert ist;
C₃-C₆-Cycloalkyl, das mit 0-3 R³¹ substituiert ist;
R³¹ aus einer oder mehreren der folgenden Gruppen ausgewählt ist:
Keto, Halogen, Cyano, -CH₂NR¹³R¹⁴, -NR¹³R¹⁴, -CO₂R¹³, -OC(=O)R¹³, -OR¹³, C₂-C₄-Alkoxyalkyl, -S(O)ₘR¹³, C₁-C₄-Alkyl, C₂-C₄-Alkenyl, C₃-C₆-Cycloalkyl;
einem carbocyclischen C₅-C₁₄-Rest, der mit 0-3 R¹² substituiert ist;
Aryl, das mit 0-3 R³² substituiert ist;
einem heterocyclischen Ringsystem, das mit 0-2 R³² substituiert ist, bestehend aus 5 bis 10 Atomen, die wenigstens ein Stickstoff-, Sauerstoff- oder Schwefelatom einschließen;
R³² aus einer oder mehreren der folgenden Gruppen ausgewählt ist, wenn es ein Substituent am Kohlenstoff ist:
Phenyl, Benzyl, Phenethyl, Phenoxy, Benzyloxy, Halogen, Hydroxy, Nitro, Cyano, C₁-C₄-Alkyl, C₃-C₆-Cycloalkyl, C₃-C₆-Cycloalkylmethyl, C₇-C₁₀-Arylalkyl, C₁-C₄-Alkoxy, -CO₂H, Hydroxamsäure, Hydrazid, Oxim, Boronsäure, Sulfonamid, Formyl, C₃-C₆-Cycloalkoxy, -OR¹³, C₁-C₄-Alkyl, das mit -NR¹³R¹⁴ substituiert ist, -NR¹³R¹⁴, C₂-C₆-Alkoxyalkyl, C₁-C₄-Hydroxyalkyl, Methylendioxy, Ethylendioxy, C₁-C₄-Haloalkyl, C₁-C₄-Haloalkoxy, C₁-C₄-Alkoxycarbonyl, C₁-C₄-Alkylcarbonyloxy, C₁-C₄-Alkylcarbonyl, C₁-C₄-Alkylcarbonylamino, -S(O)ₘR¹³, -SO₂NR¹³R¹⁴, -NHSO₂R¹⁴, -C(R¹⁴)=N(OR¹⁴);
einem fünf- oder sechsgliedrigen heterocyclischen Ring, der 1 bis 4 Heteroaotme enthält, die aus Sauerstoff, Stickstoff und Schwefel ausgewählt sind;
oder R³² eine drei - oder viergliedrige Kohlenstoffkette sein kann, die an benachbarte Kohlenstoffe am Ring gebunden ist, um einen anellierten fünf- oder sechsgliedrigen Ring zu bilden, wobei der fünf- oder sechsgliedrige Ring gegebenenfalls mit Halogen, C₁-C₄-Alkyl, C₁-C₄-Alkoxy, Hydroxy oder -NR¹³R¹⁴ an den aliphatischen Kohlenstoffen substituiert ist;
oder wenn R³² an ein gesättigtes Kohlenstoffatom gebunden ist, es Carbonyl oder Thiocarbonyl sein kann;
R³² aus einer oder mehreren der folgenden Gruppen ausgewählt ist, wenn es ein Substituent am Stickstoff ist:
Phenyl, Benzyl, Phenethyl, Hydroxy, C₁-C₄-Hydroxyalkyl, C₁-C₄-Alkoxy, C₁-C₄-Alkyl, C₃-C₆-Cycloalkyl, C₃-C₆-Cycloalkylmethyl, -CH₂NR¹³R¹⁴, -NR¹³R¹⁴, C₂-C₆-Alkoxyalkyl, C₁-C₄-Haloalkyl, C₁-C₄-Alkoxycarbonyl, C₁-C₄-Alkylcarbonyloxy, C₁-C₄-Alkylcarbonyl, -CO₂H, -C(R¹⁴)=N(OR¹⁴);
mit der Maßgabe, dass:
R⁴, R^{4A}, R⁷ und R^{7A} nicht alle Wasserstoff sind;
wenn R⁴ und R^{4A} beide Wasserstoff sind, wenigstens eines von R²² und R²³ kein Wasserstoff ist.

3. Verbindung gemäß Anspruch 1, welche die Formel (II): 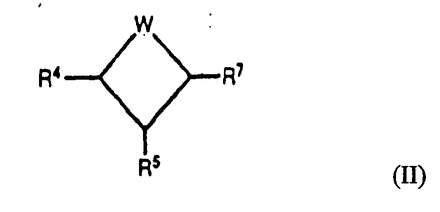 aufweist, oder ein pharmazeutisch annehmbares Salz oder eine Medikamentenvorstufe derselben, wobei
R⁴ und R⁷ unabhängig voneinander aus den folgenden Gruppen ausgewählt sind:
Wasserstoff,
C₁-C₄-Alkyl, das mit 0-3 R¹¹ substituiert ist;
C₃-C₄-Alkenyl, das mit 0-3 R¹¹ substituiert ist;
R⁵ -OR²⁰ ist;
R²⁰ wie im Anspruch 1 definiert ist;
R¹¹ aus einer oder mehreren der folgenden Gruppen ausgewählt ist:
Keto, Halogen, -CH₂NR¹³R¹⁴, -NR¹³R¹⁴, -OR¹³, C₂-C₄-Alkoxyalkyl, C₁-C₄-Alkyl, C₂-C₄-Alkenyl, C₃-C₆-Cycloalkyl;
Aryl, das mit 0-3 R¹² substituiert ist;
einem heterocyclischen Ringsystem, das mit 0-2 R¹² substituiert ist, bestehend aus 5 bis 10 Atomen, die wenigstens ein Stickstoff-, Sauerstoff- oder Schwefelatom einschließen;
R¹² aus einer oder mehreren der folgenden Gruppen ausgewählt ist, wenn es ein Substituent am Kohlenstoff ist:
Phenyl, Benzyl, Phenethyl, Phenoxy, Benzyloxy, Halogen, C₁-C₄-Alkyl, C₇-C₁₀-Arylalkyl, C₁-C₄-Alkoxy, -CO₂H, Hydroxamsäure, Hydrazid, Oxim, Boronsäure, Sulfonamid, Formyl, C₃-C₆-Cycloalkoxy, -OR¹³, C₁-C₄-Alkyl, das mit -NR¹³R¹⁴ substituiert ist, -NR¹³R¹⁴, Methylendioxy, C₁-C₄-Haloalkyl, C₁-C₄-Alkylcarbonyl, C₁-C₄-Alkylcarbonylamino, Hydroxy, Hydroxymethyl; oder
einem fünf- oder sechsgliedrigen heterocyclischen Ring, der 1 bis 4 Heteroaotme enthält, die aus Sauerstoff, Stickstoff und Schwefel ausgewählt sind;
R¹² aus Benzyl oder Methyl ausgewählt wird, wenn es ein Substituent am Stickstoff ist;
R¹³ H, C₁-C₂-Alkyl oder C₃-C₆-Aloxyalkyl ist;
R¹⁴ OH, H, C₁-C₂-Alkyl ist;
R¹³ und R¹⁴ sich alternativ verbinden können, um -(CH₂)₄-, -(CH₂)₅-, -CH₂CH₂N(R¹⁵)CH₂CH₂- oder -CH₂CH₂OCH₂CH₂- zu bilden;
W -N(R²²)C(=Z)N(R²³)- ist;
wobei Z O, S oder N-CN ist;
R²² und R²³ unabhängig voneinander aus den folgenden Gruppen ausgewählt sind:
Wasserstoff,
C₁-C₄-Alkyl, das mit 0-3 R³¹ substituiert ist;
C₃-C₄-Alkenyl, das mit 0-3 R³¹ substituiert ist;
R³¹ aus einer oder mehreren der folgenden Gruppen ausgewählt ist:
Keto, Halogen, -CH₂NR¹³R¹⁴, -NR¹³R¹⁴, -OR¹³, C₂-C₄-Alkoxyalkyl, C₁-C₄-Alkyl, C₂-C₄-Alkenyl, C₃-C₆-Cycloalkyl;
Aryl, das mit 0-3 R³² substituiert ist; oder
einem heterocyclischen Ringsystem, das mit 0-2 R³² substituiert ist, bestehend aus 5 bis 10 Atomen, die wenigstens ein Stickstoff-, Sauerstoff- oder Schwefelatom einschließen;
R³² aus einer oder mehreren der folgenden Gruppen ausgewählt ist, wenn es ein Substituent am Kohlenstoff ist:
Phenyl, Benzyl, Phenethyl, Phenoxy, Benzyloxy, Halogen, C₁-C₄-Alkyl, C₇-C₁₀-Arylalkyl, C₁-C₄-Alkoxy, -CO₂H, Hydroxamsäure, Hydrazid, Oxim, Boronsäure, Sulfonamid, Formyl, C₃-C₆-Cycloalkoxy, -OR¹³, C₁-C₄-Alkyl, das mit -NR¹³R¹⁴ substituiert ist, -NR¹³R¹⁴, Methylendioxy, C₁-C₄-Haloalkyl, C₁-C₄-Alkylcarbonyl, C₁-C₄-Alkylcarbonylamino, Hydroxy, Hydroxymethyl, -C(R¹⁴)=N(OR¹⁴);
einem fünf- oder sechsgliedrigen heterocyclischen Ring, der 1 bis 4 Heteroaotme enthält, die aus Sauerstoff, Stickstoff und Schwefel ausgewählt sind;
R³² Benzyl oder Methyl ist, wenn es ein Substituent am Stickstoff ist;
mit der Maßgabe, dass:
wenn R⁴ Wasserstoff ist, R⁷ kein Wasserstoff ist;
wenn R⁴ Wasserstoff ist, wenigstens eines von R²² und R²³ kein Wasserstoff ist.

4. Verbindung gemäß Anspruch 1 der Formel (III): 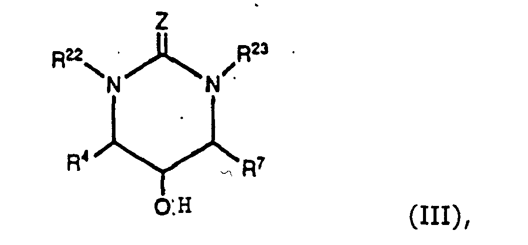 wobei
R⁴ und R⁷ unabhängig voneinander aus den folgenden Gruppen ausgewählt sind:
Wasserstoff,
C₁-C₃-Alkyl, das mit 0-3 R¹¹ substituiert ist;
R¹¹ aus einer oder mehreren der folgenden Gruppen ausgewählt ist:
Halogen, -OR¹³, C₁-C₄-Alkyl, C₃-C₅-Cycloalkyl;
Aryl, das mit 0-2 R¹² substituiert ist;
einem heterocyclischen Ringsystem, das aus Pyridyl, Pyrimidinyl, Triazinyl, Furanyl, Thienyl, Pyrrolyl, Pyrazolyl, Imidazolyl, Tetrazolyl, Benzofuranyl, Indolyl, Chinolinyl, Isochinolinyl ausgewählt ist;
R¹² aus einer oder mehreren der folgenden Gruppen ausgewählt ist, wenn es ein Substituent am Kohlenstoff ist:
Benzyloxy, Halogen, Methyl, C₁-C₄-Alkoxy, CF₃, 2-(1-Morpholino)ethoxy, -CO₂H, Hydroxamsäure, Hydrazid, Oxim, Cyano, Boronsäure, Sulfonamid, Formyl, C₃-C₆-Cycloalkoxy, C₁-C₄-Alkyl, das mit -NR¹³R¹⁴ substituiert ist, -NR¹³R¹⁴, Hydroxy, Hydroxymethyl; oder
R¹² Methyl ist, wenn es ein Substituent am Stickstoff ist;
R¹³ H oder Methyl ist;
R¹⁴ OH, H oder Methyl ist;
R¹³ und R¹⁴ sich alternativ verbinden können, um -(CH₂)₄-, -(CH₂)₅-, -CH₂CH₂N(R¹⁵)CH₂CH₂- oder -CH₂CH₂OCH₂CH₂- zu bilden;
R²² und R²³ unabhängig voneinander aus den folgenden Gruppen ausgewählt sind:
Wasserstoff,
C₁-C₄-Alkyl, das mit 0-1 R³¹ substituiert ist;
C₃-C₄-Alkenyl, das mit 0-1 R³¹ substituiert ist;
R³¹ aus einer oder mehreren der folgenden Gruppen ausgewählt ist:
Halogen, -OR¹³, C₁-C₄-Alkyl, C₃-C₅-Cycloalkyl;
Aryl, das mit 0-2 R³² substituiert ist;
einem heterocyclischen Ringsystem, das aus Pyridyl, Pyrimidinyl, Triazinyl, Furanyl, Thienyl, Pyrrolyl, Pyrazolyl, Imidazolyl, Tetrazolyl, Benzofuranyl, Indolyl, Chinolinyl, Isochinolinyl ausgewählt ist;
R³² aus einer oder mehreren der folgenden Gruppen ausgewählt ist, wenn es ein Substituent am Kohlenstoff ist:
Benzyloxy, Halogen, Methyl, C₁-C₄-Alkoxy, CF₃, 2-(1-Morpholino)ethoxy, -CO₂H, Hydroxamsäure, Hydrazid, Oxim, Cyano, Boronsäure, Sulfonamid, Formyl, C₃-C₆-Cycloalkoxy, C₁-C₄-Alkyl, das mit -NR¹³R¹⁴ substituiert ist, -NR¹³R¹⁴, Hydroxy, Hydroxymethyl, -C(R¹⁴)=N(OR¹⁴); oder
R³² Methyl ist, wenn es ein Substituent am Stickstoff ist;
mit der Maßgabe, dass:
wenn R⁴ Wasserstoff ist, R⁷ kein Wasserstoff ist;
wenn R⁴ Wasserstoff ist, wenigstens eines von R²² und R²³ kein Wasserstoff ist.

5. Verbindung gemäß Anspruch 1 der Formel (IV): 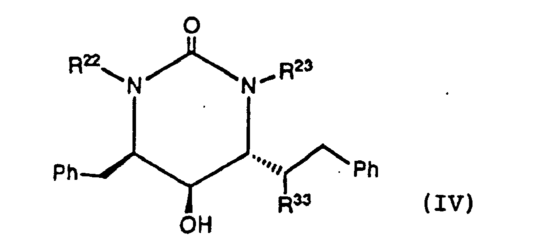 oder ein pharmazeutisch annehmbares Salz oder eine Medikamentenvorstufe derselben, wobei
R³³ OH, Halogen, H, N₃ ist oder alternativ dazu zusammen mit R²³ gefasst werden kann, um eine direkte Bindung zu bilden;
R²² und R²³ unabhängig voneinander aus:
Wasserstoff, Allyl, Propyl, Cyclopropylmethyl, n-Butyl, Isobutyl, CH₂CH=CH(CH₃)₂, Pyridylmethyl, Methallyl, n-Pentyl, Isopentyl, Hexyl, Benzyl, Pyridylmethyl, Isoprenyl, Propargyl, Picolinyl, Methoxyethyl, Cyclohexylmethyl, Dimethylbutyl, Ethoxyethyl, Methyloxazolinylmethyl, Naphthylmethyl, Methyloxazolinylmethyl, Vinyloxyethyl, Pentafluorbenzyl, Chinolinylmethyl, Carboxybenzyl, Chlorthienyl, Picolinyl, Benzyloxybenzyl, Phenylbenzyl, Adamantylethyl, Cyclopropylmethoxybenzyl, Ethoxybenzyl, Hydroxybenzyl, Hydroxymethylbenzyl, Aminobenzyl, Formylbenzyl, Cyanobenzyl, Cinnamyl, Allyloxybenzyl, Fluorbenzyl, Cyclobutylmethyl, Formaldoximbenzyl, Cyclopentylmethyl, Nitrobenzyl, Nitrilobenzyl, Carboxamidobenzyl, Carbomethoxybenzyl und Dimethylallyl ausgewählt sind.

6. Verwendung einer Verbindung gemäß irgendeinem der Ansprüche 1 bis 5 zur Herstellung eines Medikaments für die Behandlung von Vireninfektionen.

7. Pharmazeutische Zusammensetzung, die einen pharmazeutisch annehmbaren Träger und wenigstens eine Verbindung gemäß irgendeinem der Ansprüche 1 bis 5 umfasst.

## Revendications

1. Composé de la formule (I): 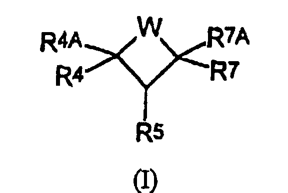 ou sel pharmaceutiquement acceptable ou forme de promédicament de celui-ci dans laquelle:
R⁴ et R⁷ sont indépendamment choisis parmi les groupes suivants:
un hydrogène;
un groupe akyle C₁-C₈ substitué avec 0-3 R¹¹;
un groupe alcényle C₂-C₈ substitué avec 0-3 R¹¹;
un groupe alcynyle C₂-C₈ substitué avec 0-3 R¹¹;
un groupe cycloalkyle C₃-C₈ substitué avec 0-3 R¹¹;
un groupe bicycloalkyle C₆-C₁₀ substitué avec 0-3 R¹¹;
un groupe aryle substitué avec 0-3 R¹²;
un résidu carbocyclique C₆-C₁₄ substitué avec 0-3 R¹²;
un système d'anneau hétérocyclique subsbtué avec 0-2 R¹², composé de 5 à 10 atomes incluant au moins un atome d'azote, d'oxygène ou de soufre;
R^{4A} et R^{7A} sont indépendamment choisis parmi les groupes suivants:
un hydrogène;
un groupe akyle C₁-C₄ substitué avec un halogène ou un groupe alkoxy C₁-C₂;
un groupe benzyle substitué avec un halogène ou un groupe alkoxy C₁-C₂;
R⁴ et R^{4A} peuvent selon une variante se joindre pour former un anneau carbocyclique de 5-7 membres substitué avec 0-2 R¹²;
R⁷ et R^{7A} peuvent selon une variante se joindre pour former un anneau carbocyclique de 5-7 membres substitué avec 0-2 R¹²;
R⁵ est choisi parmi un groupe fluoio, difluoro, =O et -OR²⁰;
R²⁰ est choisi parmi:
un hydrogène;
un groupe alkyle C₁-C₆ substitué avec 0-3 R¹¹;
un groupe alkoxyalkyle C₃-C₆ substitué avec 0-3 R¹¹;
un groupe alkylcarbonyle C₁-C₆ substitué avec 0-3 R¹¹;
un groupe alkoxycarbonyle C₁-C₆ substitué avec 0-3 R¹¹;
un groupe benzoyle substitué avec 0-3 R¹²;
un groupe phénoxycarbonyle substitué avec 0-3 R¹²;
un groupe phénylaminocarbonyle substitué avec 0-3 R¹²;
R¹¹ est choisi parmi un ou plusieurs des groupes suivants:
céto, halogène, cyano, -CH₂NR¹³R¹⁴, -NR¹³R¹⁴, -CO₂R¹³, -OC(=O)R¹³, -OR¹³, alkoxyalkyle C₂-C₆, -S(O)ₘR¹³, -NHC(=NH)NHR¹³, -C(=NH)NHR¹³, -C(=O)NR¹³R¹⁴, -NR¹⁴C(=O)R¹³, -NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, alkyle C₁-C₄, alcényle C₂-C₄, cycloalkyle C₃-C₆, cycloalkylméthyle C₃-C₆;
1-3 acides aminés, liés ensemble par l'intermédiaire de liaisons amides et liés à R⁴ ou R⁷ par l'intermédiaire de l'extrémité amine ou carboxylate;
un résidu carbocyclique C₅-C₁₄ substitué avec 0-3 R¹²;
un groupe aryle substitué avec 0-3 R¹²;
un système d'anneau hétérocyclique substitué avec 0-2 R¹², composé de 5 à 10 atomes incluant au moins un atome d'azote, d'oxygène ou de soufre;
R¹², lorsque c'est un substituant sur un carbone, est choisi parmi un ou plusieurs des groupes suivants;
phényle, benzyle, phénéthyle, phénoxy, benzyloxy, halogène, hydroxyle, nitro, cyano, alkyle C₁-C₄, cycloalkyle C₃-C₆, cycloalkylméthyle C₃-C₆, arylalkyle C₇-C₁₀, alkoxy C₁-C₄, -CO₂H, acide hydroxamique, hydrazide, oxime, acide boronique, sulfonamide, formyle, cycloalkoxy C₃-C₆, -OR¹³, alkyle C₁-C₄ substitué avec -NR¹³R¹⁴, -NR¹³R¹⁴, alkoxyalkyle C₂-C₆, hydroxyalkyle C₁-C₄, métylènedioxy, éthylènedioxy, haloalkyle C₁-C₄, haloalkoxy C₁-C₄, alkoxycarbonyle C₁-C₄, alkylcarbonyloxy C₁-C₄, alkylcarbonyle C₁-C₄, alkylcarbonylamino C₁-C₄, -S(O)ₘR¹³, -SO₂NR¹³R¹⁴, -NHSO₂R¹⁴, -OCH₂CO₂H, 2-(1-morpholino)éthoxy;
un anneau hétérocyclique de 5 ou 6 membres contenant de 1 à 4 hétéroatomes choisis parmi un oxygène, un azote et un soufre;
ou R¹² peut être une chaîne de 3 ou 4 carbones attachée à des carbones adjacents sur l'anneau pour former un anneau condensé de 5 ou 6 membres, ledit anneau de 5 ou 6 membres étant éventuellement substitué sur les carbones aliphatiques avec un halogène, un groupe alkyle C₁-C₄, alkoxy C₁-C₄, hydroxyle ou -NR¹³R¹⁴; ou, lorsque R¹² est attaché à un atome de carbone saturé, il peut être un groupe carbonyle ou thiocarbonyle;
R¹², lorsque c'est un substituant sur un azote, est choisi parmi un ou plusieurs des groupes suivants:
phényle, benzyle, phénéthyle, hydroxyle, hydroxyalkyle C₁-C₄, alkoxy C₁-C₄, alkyle C₁-C₄, cycloalkyle C₃-C₆, cycloalkylméthyle C₃-C₆, -CH₂NR¹³R¹⁴, -NR¹³R¹⁴, alkoxyalkyle C₂-C₆, haloalkyle C₁-C₄, alkoxycarbonyle C₁-C₄, -CO₂H, alkylcarbonyloxy C₁-C₄, alkylcarbonyle C₁-C₄;
R¹³ est H, un groupe phényle, benzyle, alkyle C₁-C₆ ou alkoxyalkyle C₃-C₆;
R¹⁴ est OH, H, un groupe alkyle C₁-C₄ ou benzyle;
R¹³ et R¹⁴ peuvent selon une variante se joindre pour former -(CH₂)₄-, -(CH₂)₅-, -CH₂CH₂N(R¹⁵)CH₂CH₂- ou -CH₂CH₂OCH₂CH₂-;
R¹⁵ est H ou CH₃;
m est 0, 1 ou 2;
W est choisi parmi:
-N(R²²)C(=Z) N(R²³)-;
-N(R²²)S(=O)N(R²³)-;
dans lesquelles formules:
Z est O, S Ou NR²⁴;
R²² et R²³ sont indépendamment choisis parmi ce qui suit:
un hydrogène;
un groupe alkyle C₁-C₈ substitué avec 0-3 R³¹;
un groupe alcényle C₂-C₈ substitué avec 0-3 R³¹;
un goupe alcynyle C₂-C₈ substitué avec 0-3 R³¹;
un groupe cycloalkyle C₃-C₈ substitué avec 0-3 R³¹,
un groupe bicycloalkyle C₆-C₁₀ substitué avec 0-3 R³¹;
un groupe aryle substitué avec 0-3 R³²;
un résidu carbocyclique C₆-C₁₄ substitué avec 0-3 R³²;
un système d'anneau hétérocyclique substitué avec 0-2 R³², composé de 5 à 10 atomes incluant au moins un atome d'azote, d'oxygène ou de soufre;
R²⁴ est choisi parmi: un groupe hydroxyle; amino, alkyle C₁-C₄; alkoxy C₁-C₄; aminoalkyle C₁-C₄; cyano; nitro; benzyloxy;
selon une variante, R²² peut se joindre avec R⁴ ou R^{4A} pour former un anneau hétéocyclique ou carbocyclique condensé de cinq ou six membres substitué avec 0-2 R¹²; et
selon une variante, R²³ peut se joindre avec R⁷ ou R^{7A} pour former un anneau hétérocyclique ou carbocyclique condensé de cinq ou six membres substitué avec 0-2 R¹²; et
selon une variante, W peut se joindre avec R⁵ pour former un anneau hétérocyclique ou carbocyclique condensé de trois à sept membres substitué avec 0-2 R¹²;
R³¹ est choisi parmi un ou plusieurs des groupes suivants:
céto, halogène, cyano, -CH₂NR¹³R¹⁴, -NR¹³R¹⁴, -CO₂R¹³, -OC(=O)R¹³, -OR¹³, alkoxyalkyle C₂-C₆, -S(O)ₘR¹³, -NHC(=NH)NHR¹³, -C(=NH)NHR¹³, -C(=O)NR¹³R¹⁴, -NR¹⁴C(=O)R¹³, =NOR¹⁴, -NR¹⁴C(=O)OR¹⁴, -OC(=O)NR¹³R¹⁴, -NR¹³C(=O)NR¹³R¹⁴, -NR¹⁴SO₂NR¹³R¹⁴, -NR¹⁴SO₂R¹³, -SO₂NR¹³R¹⁴, alkyle C₁-C₄, alcényle C₂-C₄, cycloalkyle C₃-C₆, cycloalkylméthyle C₃-C₆;
1-3 acides aminés, liés ensemble par l'intermédiaire de liaisons amides et liés à R⁴ ou R⁷ par l'intermédiaire de l'extrémité amine ou carboxylate;
un résidu carbocyclique C₅-C₁₄ substitué avec 0-3 R³²;
un groupe aryle substitué avec 0-3 R³²; ou
un système d'anneau hétérocyclique substitué avec 0-2 R³², composé de 5 à 10 atomes incluant au moins un atome d'azote, d'oxygene ou de soufre;
R³², lorsque c'est un substituant sur un carbone, est choisi parmi un ou plusieurs des groupes suivants:
phényle, benzyle, phénéthyle, phénoxy, benzyloxy, halogène, hydroxyle, nitro, cyano, alkyle C₁-C₄, cycloalkyle C₃-C₆, cycloalkylméthyle C₃-C₆, arylalkyle C₇-C₁₀, alkoxy C₁-C₄, -CO₂H, acide hydroxamique, hydrazide, oxime, acide boronique, sulfonamide, formyle, cycloalkoxy C₃-C₆, -OR¹³, alkyle C₁-C₄ substitué avec -NR¹³R¹⁴, -NR¹³R¹⁴, alkoxyalkyle C₂-C₆, hydroxyalkyle C₁-C₄, méthylènedioxy, éthylènedioxy, haloalkyle C₁-C₄, haloalkoxy C₁-C₄, alkoxycarbonyle C₁-C₄, alkylcarbonyloxy C₁-C₄, alkylcarbonyle C₁-C₄, alkylcarbonylamino C₁-C₄, -S(O)ₘR¹³, -SO₂NR¹³R¹⁴, -NHSO₂R¹⁴, -OCH₂CO₂H, 2-(1-morpholino)éthoxy, -C(R¹⁴)=N(OR¹⁴); ou
un anneau hétérocyclique de 5 ou 6 membres contenant de 1 à 4 hetéroatomes choisis parmi un oxygène, un azote et un soufre;
ou R³² peut être une chaîne de 3 ou 4 carbones attachée à des carbones adjacents sur l'anneau pour former un anneau condensé de 5 ou 6 membres, ledit anneau de 5 ou 6 membres étant éventuellement substitué sur les carbones aliphatiques avec un halogène, un groupe alkyle C₁-C₄, alkoxy C₁-C₄, hydroxyle ou -NR¹³R¹⁴; ou, lorsque R³² est attaché à un atome de carbons saturé, il peut être un goupe carbonyle ou thiocarbonyle;
R³², lorsque c'est un substituant sur un azote, est choisi parmi un ou plusieurs des groupes suivants:
phényle, benzyle, phénéthyle, hydroxyle, hydroxyalkyle C₁-C₄, alkoxy C₁-C₄, alkyle C₁-C₄, cycloalkyle C₃-C₆, cycloalkylméthyle C₃-C₆, -CH₂NR¹³R¹⁴, -NR¹³R¹⁴, alkoxyalkyle C₂-C₆, haloalkyle C₁-C₄, alkoxycarbonyle C₁-C₄, -CO₂H, alkylcarbonyloxy C₁-C₄, alkylcarbonyle C₁-C₄, -C(R¹⁴)=N(OR¹⁴);
sous réserve que:
R⁴, R^{4A}, R⁷ et R^{7A} ne soient pas tous des hydrogènes;
lorsque R⁴ et R^{4A} sont det hydrogènes, au moins un parmi R²² et R²³ ne soit pas un hydrogène.

2. Composé suivant la revendication 1, dans lequel:
R⁴ et R⁷ sont indépendamment choisis parmi les groupes suivants:
un hydrogène;
un groupe alkyle C₁-C₄ substitué avec 0-3 R¹¹;
un groupe alcényle C₃-C₄ substitué avec 0-3 R¹¹;
un groupe alcynyle C₃-C₄ substitué avec 0-3 R¹¹;
R^{4A} et R^{7A} sont des hydrogènes;
R²⁰ est choisi parmi:
un hydrogène;
un groupe alkylcarbonyle C₁-C₆;
un groupe alkoxycarbonyle C₁-C₆;
un goupe benzoyle;
R¹¹ est choisi parmi un ou plusieurs des groupes suivants:
céto, halogène, cyano, -CH₂NR¹³R¹⁴, -NR¹³R¹⁴, -CO₂R¹³, -OC(=O)R¹³, -OR¹³, alkoxyalkyle C₂-C₄, -S(O)ₘR¹³, alkyle C₁-C₄, alcényle C₂-C₄, cycloalkyle C₃-C₆;
un résidu carbocyclique C₅-C₁₄ substitué avec 0-3 R¹²;
un groupe aryle substitué avec 0-3 R¹²;
un système d'anneau hétérocyclique substitué avec 0-2 R¹², composé de 5 à 10 atomes incluant au moins un atome d'azote, d'oxygène ou de soufre;
R¹², lorsque c'est un substituant sur un carbone, est choisi parmi un ou plusieurs des groupes suivants:
phényle, benzyle, phénéthyle, phénoxy, benzyloxy, halogène, hydroxyle, nitro, cyano, alkyle C₁-C₄, cycloalkyle C₃-C₆, cycloalkylméthyle C₃-C₆, arylalkyle C₇-C₁₀, alkoxy C₁-C₄, -CO₂H, acide hydroxamique, hydrazide, oxime, acide boronique, sulfonamide, formyle, cycloalkoxy C₃-C₆, -OR¹³, alkyle C₁-C₄ substitué avec -NR¹³R¹⁴, -NR¹³R¹⁴, alkoxyalkyle C₂-C₆, hydroxyalkyle C₁-C₄, méthylènedioxy, éthylènedioxy, haloalkyle C₁-C₄, haloalkoxy C₁-C₄, alkoxycarbonyle C₁-C₄, alkylcarbonyloxy C₁-C₄, alkylcarbonyle C₁-C₄, alkylcarbonylamino C₁-C₄, -S(O)ₘR¹³, -SO₂NR¹³R¹⁴, -NHSO₂R¹⁴; ou
un anneau hétérocyclique de 5 ou 6 membres contenant de 1 à 4 hétéroatomes choisis parmi un oxygène, un azote ou un soufre;
ou R¹² peut être une chaîne de 3 ou 4 carbones attachée à des carbones adjacents sur l'anneau pour former un anneau condensé de 5 ou 6 membres, ledit anneau de 5 ou 6 membres étant éventuellement substitué sur les carbones aliphatiques avec un halogène, un groupe alkyle C₁-C₄, alkoxy C₁-C₄, hydroxyle ou -NR¹³R¹⁴; ou, lorsque R¹² est attaché à un atome de carbone saturé, il peut être un groupe carbonyle ou thiocarbonyle;
R¹², lorsque c'est un substituant sur un azote, est choisi parmi un ou plusieurs des groupes suivants:
phényle, benzyle, phénéthyle, hydroxyle, hydroxyalkyle C₁-C₄, alkoxy C₁-C₄, alkyle C₁-C₄, cycloalkyle C₃-C₆, cycloalkylméthyle C₃-C₆, -CH₂NR¹³R¹⁴, -NR¹³R¹⁴, alkoxyalkyle C₂-C₆, haloalkyle C₁-C₄, alkoxycarbonyle C₁-C₄, alkylcarbonyloxy C₁-C₄, alkylcarbonyle C₁-C₄, -CO₂H;
R¹³ est H, un groupe alkyle C₁-C₆ ou alkoxyalkyle C₃-C₆;
R¹⁴ est OH, H, un groupe alkyle C₁-C₄ ou benzyle;
R¹³ et R¹⁴ peuvent selon une variante se joindre pour former -(CH₂)₄-, -(CH₂)₅-, -CH₂CH₂N(R¹⁵)CH₂CH₂-, ou -CH₂CH₂OCH₂CH₂-;
R¹⁵ est H ou CH₃;
m est 0, 1 ou 2;
W est:
-N(R²²)C(=Z)N(R²³)-;
dans laquelle formule:
Z est O, S, N-CN, N-OH, N-OCH₃;
R²² et R²³ sont indépendamment choisis parmi ce qui suit:
un hydrogène;
un groupe alkyle C₁-C₈ substitué avec 0-3 R³¹;
un groupe alcényle C₃-C₈ substitué avec 0-3 R³¹;
un groupe alcynyle C₃-C₈ substitué avec 0-3 R³¹;
un groupe cycloalkyle C₃-C₆ substitué avec 0-3 R³¹;
R³¹ est choisi parmi un ou plusieurs des groupes suivants:
céto, halogène, cyano, -CH₂NR¹³R¹⁴, -NR¹³R¹⁴, -CO₂R¹³, -OC(=O)R¹³, -OR¹³, alkoxyalkyle C₂-C₄, -S(O)ₘR¹³, alkyle C₁-C₄, alcényle C₂-C₄, cycloalkyle C₃-C₆;
un résidu carbocyclique C₅-C₁₄ substitué avec 0-3 R¹²;
un groupe aryle substitué avec 0-3 R³²;
un système d'ameau hétérocyclique substitué avec 0-2 R³², composé de 5 à 10 atomes incluant au moins un atome d'azote, d'oxygène ou de soufre;
R³², lorsque c'est un substituant sur un carbone, est choisi parmi un ou plusieurs des groupes suivants:
phényle, benzyle, phénéthyle, phénoxy, benzyloxy, halogène, hydroxyle, nitro, cyano, alkyle C₁-C₄, cycloalkyle C₃-C₆, cycloalkylméthyle C₃-C₆, arylalkyle C₇-C₁₀, alkoxy C₁-C₄, -CO₂H, acide hydroxamique, hydrazide, oxime, acide boronique, sulfonamide, formyle, cycloalkoxy C₃-C₆, -OR¹³, alkyle C₁-C₄ substitué avec -NR¹³R¹⁴, -NR¹³R¹⁴, alkoxyalkyle C₂-C₆, hydroxyalkyle C₁-C₄, méthylènedioxy, éthylènedioxy, haloalkyle C₁-C₄, haloalkoxy C₁-C₄, alkoxycarbonyle C₁-C₄, alkylcarbonyloxy C₁-C₄, alkylcarbonyle C₁-C₄, alkylcarbonylamino C₁-C₄, -S(O)ₘR¹³, -SO₂NR¹³R¹⁴, -NHSO₂R¹⁴, -C(R¹⁴)=N(OR¹⁴);
un anneau hétérocyclique de 5 ou 6 membres contenant de 1 à 4 hétéroatomes choisis parmi un oxygène, un azote et un soufre;
ou R³² peut être une chaîne de 3 ou 4 carbones attachée à des carbones adjacents sur l'anneau pour former un anneau condensé de 5 ou 6 membres, ledit anneau de 5 ou 6 membres étant éventuellement substitué sur les carbones aliphatiques avec un halogène, un groupe alkyle C₁-C₄, alkoxy C₁-C₄, hydroxyle ou -NR¹³R¹⁴; ou, lorsque R³² est attaché à un atome de carbone saturé, il peut être un groupe carbonyle ou thiocarbonyle;
R³², lorsque c'est un substituant sur un azote, est choisi parmi un ou plusieurs des groupes suivants:
phényle, benzyle, phénéthyle, hydroxyle, hydroxyalkyle C₁-C₄, alkoxy C₁-C₄, alkyle C₁-C₄, cycloalkyle C₃-C₆, cycloalkylméthyle C₃-C₆, -CH₂NR¹³R¹⁴, -NR¹³R¹⁴, alkoxyalkyle C₂-C₆, haloalkyle C₁-C₄, alkoxycarbonyle C₁-C₄, alkylcarbonyloxy C₁-C₄, alkylcarbonyle C₁-C₄, -CO₂H, -C(R¹⁴)=N(OR¹⁴);
sous réserve que:
R⁴, R^{4A}, R⁷ et R^{7A} ne soient pas tous des hydrogènes;
lorsque R⁴ et R^{4A} sont deux hydrogènes, au moins un parmi R²² et R²³ ne soit pas un hydrogène.

3. Composé suivant la revendication 1, présentant la formule (II); 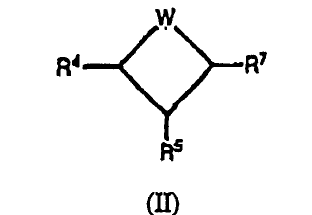 ou sel pharmaceutiquement acceptable ou forme de promédicament de celui-ci dans laquelle:
R⁴ et R⁷ sont indépendamment choisis parmi les groupes suivants:
un hydrogène;
un groupe alkyle C₁-C₄ substitué avec 0-3 R¹¹;
un groupe alcényle C₃-C₄ substitué avec 0-3 R¹¹;
R⁵ est -OR²⁰;
R²⁰ est comme il est défini dans la revendication 1;
R¹¹ est choisi parmi un ou plusieurs des groupes suivants:
céto, halogène, -CH₂NR¹³R¹⁴, -NR¹³R¹⁴, -OR¹³, alkoxyalkyle C₂-C₄, alkyle C₁-C₄, alcényle C₂-C₄, cycloalkyle C₃-C₆;
un groupe aryle substitué avec 0-3 R¹²;
un système d'anneau hétérocyclique substitué avec 0-2 R¹², composé de 5 à 10 atomes incluant au moins un atome d'azote, d'oxygène ou de soufre;
R¹², lorsque c'est un substituant sur un carbone, est choisi parmi un ou plusieurs des groupes suivants:
phényle, benzyle, phénéthyle, pbénoxy, benzyloxy, halogène, alkyle C₁-C₄, arylalkyle C₇-C₁₀, alkoxy C₁-C₄, -CO₂H, acide hydroxamique, hydrazide, oxime, acide boronique, sulfonamide, formyle, cycloalkoxy C₃-C₆, -OR¹³, alkyle C₁-C₄ substitué avec -NR¹³R¹⁴, -NR¹³R¹⁴, methylènedioxy, haloalkyle C₁-C₄, alkylcarbonyle C₁-C₄, alkylcarbonylamino C₁-C₄, hydroxyle, hydroxyméthyle; ou
un anneau hétéroeyclique de 5 ou 6 membres contenant de 1 à 4 hétéroatomes choisis parmi un oxygène, un azote ou un soufre;
R¹², lorsque c'est un substituant sur un azote, est choisi parmi un groupe benzyle ou méthyle;
R¹³ est H, un groupe alkyle C₁-C₂ ou alkoxyalkyle C₃-C₆;
R¹⁴ est OH, H ou un groupe alkyle C₁-C₂;
R¹³ et R¹⁴ peuvent selon une variante se joindre pour former -(CH₂)₄-, -(CH₂)₅-, -CH₂CH₂N(R¹⁵)CH₂CH₂- ou -CH₂CH₂OCH₂CH₂-;
W est -N(R²²)C(=Z)N(R²³)-;
dans laquelle formule:
Z est O, S ou N-CN;
R²² et R²³ sont indépendamment choisis parmi ce qui suit:
un hydrogène;
un groupe alkyle C₁-C₄ substitué avec 0-3 R³¹;
un groupe alcényle C₃-C₄ substitué avec 0-3 R³¹;
R³¹ est choisi parmi un ou plusieurs des groupes suivants:
céto, halogène, -CH₂NR¹³R¹⁴, -NR¹³R¹⁴, -OR¹³, alkoxyalkyle C₂-C₄, alkyle C₁-C₄, alcényle C₂-C₄, cycloalkyle C₃-C₆;
un groupe aryle substitué avec 0-3 R³²; ou
un système d'anneau hétérocyclique substitué avec 0-2 R³², composé de 5 à 10 atomes incluant au moins un atome d'azote, d'oxygène ou de soufre;
R³², lorsque c'est un substituant sur un carbone, est choisi parmi un ou plusieurs des groupes suivants:
phényle, benzyle, phénéthyle, phénoxy, benzyloxy, halogène, alkyle C₁-C₄, arylalkyle C₇-C₁₀, alkoxy C₁-C₄, -CO₂H, acide hydroxamique, hydrazide, oxime, acide boronique, sulfonamide, formyle, cycloalkoxy C₃-C₆, -OR¹³, alkyle C₁-C₄ substitué avec -NR¹³R¹⁴, -NR¹³R¹⁴, méthylènedioxy, haloalkyle C₁-C₄, alkylcarbonyle C₁-C₄, alkylcarbonylamino C₁-C₄, hydroxyle, hydroxyméthyle, - C(R¹⁴)=N(OR¹⁴);
un anneau hétérocyclique de 5 ou 6 membres contenant de 1 à 4 hétéroatomes choisis parmi un oxygène, un azote et un soufre;
R³², lorsque c'est un substituant sur un azote, est un goupe benzyle ou méthyle; sous réserve que:
lorsque R⁴ est un hydrogène, R⁷ ne soit pas un hydrogène;
lorsque R⁴ est un hydrogène, au moins un parmi R²² et R²³ ne soit pas un hydrogène.

4. Composé suivant la revendication 1, présentant la formule (III): 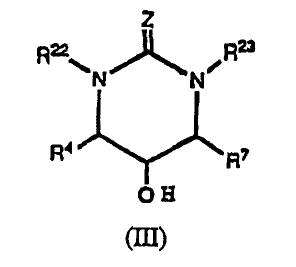 dans laquelle:
R⁴ et R⁷ sont indépendamment choisis parmi les groupes suivants:
un hydrogène;
un groupe alkyle C₁-C₃ substitué avec 0-3 R¹¹;
R¹¹ est choisi parmi un ou plusieurs des groupes suivants:
halogène, -OR¹³, alkyle C₁-C₄, cycloalkyle C₃-C₅;
un groupe aryle substitué avec 0-2 R¹²;
un système d'anneau hétérocyclique choisi parmi un groupe pyridyle, pyrimidinyle, triazinyle, furannyle, thiényle, pyrrolyle, pyrazolyle, imidazolyle, tétrazolyle, benzofurannyle, indolyle, quinoléinyle, isoquinoléinyle;
R¹², lorsque c'est un subsdtuant sur un carbone, est choisi parmi un ou plusieurs des groupes suivants:
benzyloxy, halogène, méthyle, alkoxy C₁-C₄, CF₃, 2-(1-motpholino)éthoxy, -CO₂H, acide hydroxamique, hydrazide, oxime, cyano, acide boronique, sulfonamide, formyle, cycloalkoxy C₃-C₆, alkyle C₁-C₄ substitué avec -NR¹³R¹⁴, -NR¹³R¹⁴, hydroxyle, hydroxyméthyle; ou
R¹², lorsque c'est un substituant sur un azote, est un groupe méthyle;
R¹³ est H ou un groupe méthyle;
R¹⁴ est OH, H ou un groupe méthyle;
R¹³ et R¹⁴ peuvent selon une variante se joindre pour former -(CH₂)₄-, -(CH₂)₅-,
-CH₂CH₂N(R¹⁵)CH₂CH₂- ou -CH₂CH₂OCH₂CH₂-;
R²² et R²³ sont indépendamment choisis parmi ce qui suit:
un hydrogène;
un groupe alkyle C₁-C₄ substitué avec 0-1 R³¹;
un groupe alcényle C₃-C₄ substitué avec 0-1 R³¹;
R³¹ est choisi parmi un ou plusieurs des groupes suivants:
halogène, -OR¹³, alkyle C₁-C₄, cycloalkyle C₃-C₅;
un groupe aryle substitué avec 0-2 R³²;
un système d'anneau hétérocyclique choisi parmi un groupe pyridyle, pyrimidinyle, triazinyle, furannyle, thiényle, pyrrolyle, pyrazolyle, imidazolyle, tétrazolyle, benzofurannyle, indolyle, quinoléinyle, isoquinoléinyle;
R³², lorsque c'est un substituant sur un carbone, est choisi parmi un ou plusieurs des groupes suivants:
benzyloxy, halogène, méthyle, alkoxy C₁-C₄, CF₃, 2-(1-morpholino)éthoxy, -CO₂H, acide hydroxamique, hydrazide, oxime, cyano, acide boronique, sulfonamide, formyle, cycloalkoxy C₃-C₆, alkyle C₁-C₄ substitué avec -NR¹³R¹⁴, -NR¹³R¹⁴, hydroxyle, hydroxyméthyle, -C(R¹⁴)=N(OR¹⁴); ou
R³², lorsque c'est un substatuant sur un azote, est un groupe méthyle;
sous réserve que:
lorsque R⁴ est un hydrogène, R⁷ ne soit pas un hydrogène;
lorsque R⁴ est un hydrogène, au moins un parmi R²² et R²³ ne soit pas un hydrogène.

5. Composé suivant la revendication 1, présentant la formule (IV): 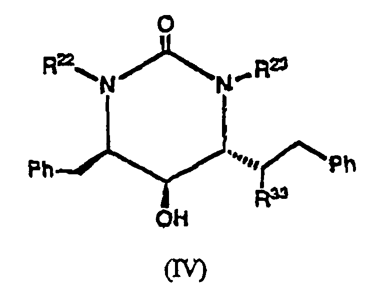 ou sel pharmaceutiquement acceptable ou forme de promédicament de celui-ci dans laquelle:
R³³ est OH, un halogène, H, N₃ ou peut selon une variante être pris ensemble avec R²³ pour former une liaison directe;
R²² et R²³ sont indépendamment choisis parmi:
un hydrogène, un groupe allyle, propyle, cyclopropylméthyle, n-butyle, i-butyle, CH₂CH=CH(CH₃)₂, pyridylmétliyle, méthallyle, n-pentyle, i-pentyle, hexyle, benzyle, pyridylméthyle, isoprényle, propargyle, picolinyle, méthoxyéthyle, cyclohexylméthyle, diméthylbutyle, éthoxyéthyle, méthyloxazolinylméthyle, naphtylméthyle, méthyloxazolinylméthyle, vinyloxyéthyle, pentafluorobenzyle, quinoléinylméthyle, carboxybenzyle, chlorothiényle, picolinyle, benzyloxybenzyle, phénylbenzyle, adamantyléthyle, cyclopropylméthoxybenzyle, éthoxybenzyle, hydroxybenzyle, hydroxyméthylbenzyle, aminobenzyle, formylbenzyle, cyanobenzyle, cinnamyle, allyloxybenzyle, fluorobenzyle, cyclobutylméthyle, formaldoximebenzyle, cyclopentylméthyle, nitrobenzyle, nitrilobenzyle, carboxamidobenzyle, carbométhoxybenzyle et diméthylallyle.

6. Utilisation d'un composé suivant l'une quelconque des revendications 1 à 5 pour la préparation d'un médicament destiné au traitement d'infections virales.

7. Composition pharmaceutique comprenant un excipient pharmaceutiquement acceptable et au moins un composé suivant l'une quelconque des revendications 1 à 5.
